# FASCICULE DE BREVET EUROPEEN

(11) **EP 1 309 860 B1**
(45) Date de publication et mention de la délivrance du brevet: **14.11.2007**
(21) Numéro de dépôt: 01956629.8
(22) Date de dépôt: 20.07.2001
(51) Int. Cl.: G01N 33/50, G01N 33/569, C07K 7/06, A61K 39/00

(54) **PROCEDE DE CRIBLAGE DE PEPTIDES UTILISABLES EN IMMUNOTHERAPIE**
VERFAHREN ZUM AUFFINDEN VON PEPTIDEN ZUR VERWENDUNG IN DER IMMUNTHERAPIE
METHOD FOR SCREENING PEPTIDES FOR USE IN IMMUNOTHERAPY

(30) Priorité: 21.07.2000 FR 0009591
(43) Date de publication de la demande: 14.05.2003
(73) Titulaire: INSTITUT NATIONAL DE LA SANTE ET DE LA RECHERCHE MEDICALE (INSERM), 75654 Paris Cédex 13 (FR); Institut Gustave Roussy (IGR), 94805 Villejuif Cedex (FR)
(72) Inventeur: KOSMATOPOULOS, Kostas, F-75013 Paris (FR); TOURDOT, Sophie, F-95270 Chaumontel (FR); SCARDINO, Antonio, F-75018 Paris (FR); GROSS, David, Alexandre, F-75020 Paris (FR)
(74) Mandataire: Vialle-Presles, Marie José
(86) Numéro de dépôt international: PCT/FR2001/002387
(87) Numéro de publication internationale: WO 2002/008716

(56) Documents cités:
- WO-A-00/02581
- WO-A-00/25813
- WO-A-91/04045
- WO-A-94/20127
- WO-A-95/27901
- WO-A-97/35193
- US-A- 5 726 023
- POGUE REBECCA R ET AL: "Amino-terminal alteration of the HLA-A*0201-restricted human immunodeficiency virus pol peptide increases complex stability and in vitro immunogenicity." PROCEEDINGS OF THE NATIONAL ACADEMY OF SCIENCES OF THE UNITED STATES, vol. 92, no. 18, 1995, pages 8166-8170, XP002170616 1995 ISSN: 0027-8424 cité dans la demande
- DATABASE BIOSIS [en ligne] BIOSCIENCES INFORMATION SERVICE, PHILADELPHIA, PA, US; 1995 HUDRISIER DENIS ET AL: "Relative implication of peptide residues in binding to major histocompatibility complex class I, H-2D-b: Application to the design of high-affinity, allele-specific peptides." Database accession no. PREV199598517001 XP002170619 cité dans la demande & MOLECULAR IMMUNOLOGY, vol. 32, no. 12, 1995, pages 895-907, ISSN: 0161-5890
- OUKKA M, MANUGUERRA JC, LIVADITIS N, TOURDOT S, RICHE N, VERGNON I, CORDOPATIS P, KOSMATOPOULOS K.: "Protection against lethal viral infection by vaccination with nonimmunodominant peptides" JOURNAL OF IMMUNOLOGY, vol. 157, no. 7, 1 octobre 1996 (1996-10-01), pages 3039-3045, XP002170617 cité dans la demande
- TOURDOT S, OUKKA M, MANUGUERRA JC, MAGAFA V, VERGNON I, RICHE N, BRULEY-ROSSET M, CORDOPATIS P, KOSMATOPOULOS K: "Chimeric peptides: a new approach to enhancing the immunogenicity of peptides with low MHC class I affinity: application in antiviral vaccination" JOURNAL OF IMMUNOLOGY, vol. 159, no. 5, 1 septembre 1997 (1997-09-01), pages 2391-2398, XP002170618 cité dans la demande
- MICHELETTI F ET AL: "Selective amino acid substitutions of a subdominant Epstein-Barr virus LMP2-derived epitope increase HLA/peptide complex stability and immunogenicity: Implications for immunotherapy of Epstein-Barr virus-associated malignancies" EUR. J. IMMUNOL., vol. 29, 1999, pages 2579-2589,
- PARKHURST M R ET AL: "Improved induction of melanoma-reactive CTL with peptides from the melanoma antigen gp100 modified at HLA-A*0201-binding residues" JOURNAL OF IMMUNOLOGY, vol. 157, no. 6, 1996, pages 2539-2548,
- RUPPERT J ET AL: "Prominent Role of Secondary Anchor Residues in Peptide Binding to HLA-A2.1 Molecules" CELL, vol. 74, 1993, pages 929-937, XP000609144
- VAN DER BURG ET AL.: JOURNAL OF IMMUNOLOGY, vol. 156, 1996, pages 3308-3314,

### LISTE DE SEQUENCES

<110> INSERM
   INSTITUT GUSTAVE ROUSSY
   KOSMATOPOULOS, Kostas
   TOURDOT, Sophie
   SCARDINO, Antonio
   GROSS, David, Alexandre
<120> PROCEDE DE CRIBLAGE DE PEPTIDES UTILISABLES EN IMMUNOTHERAPIE
<130> MJPcb598-40
<140>
   <141>
<150> FR 0009591
   <151> 2000-07-21
<160> 70
<170> PatentIn Ver. 2.1
<210> 1
   <211> 9
   <212> PRT
   <213> PEPTIDES SYNTHETIQUES
<400> 1
   Ser Met Ile Glu Asn Leu Glu Tyr Met
    1               5
<210> 2
   <211> 9
   <212> PRT
   <213> PEPTIDES SYNTHETIQUES
<220>
   <221> VARIANT
   <222> (2)
   <223> Xaa(1) = Tyr ou Lys et Xaa(4) = Glu ou Lys
<400> 2
   Xaa Ala Ile Xaa Asn Ala Glu Ala Leu
    1               5
<210> 3
   <211> 9
   <212> PRT
   <213> PEPTIDES SYNTHETIQUES
<400> 3
   Ile Val Gly Ala Glu Thr Phe Tyr Val
   1 5
<210> 4
   <211> 9
   <212> PRT
   <213> PEPTIDES SYNTHETIQUES
<400> 4
   Pro Leu Thr Ser Ile Ile Ser Ala Val
   1 5
<210> 5
   <211> 9
   <212> PRT
   <213> PEPTIDES SYNTHETIQUES
<400> 5
   Ala Leu Ile His His Asn Thr His Leu
   1 5
<210> 6
   <211> 9
   <212> PRT
   <213> PEPTIDES SYNTHETIQUES
<400> 6
   Thr Leu Glu Glu Ile Thr Gly Tyr Leu
   1 5
<210> 7
   <211> 9
   <212> PRT
   <213> PEPTIDES SYNTHETIQUES
<400> 7
   Pro Leu Gln Pro Glu Gln Leu Gln Val
   1 5
<210> 8
   <211> 9
   <212> PRT
   <213> PEPTIDES SYNTHETIQUES
<400> 8
   Glu Met Met Thr Ala Cys Gln Gly Val
   1 5
<210> 9
   <211> 10
   <212> PRT
   <213> PEPTIDES SYNTHETIQUES
<400> 9
   Leu Leu Asp Thr Gly Ala Asp Asp Thr Val
   1 5 10
<210> 10
   <211> 9
   <212> PRT
   <213> PEPTIDES SYNTHETIQUES
<400> 10
   Arg Leu Phe Phe Tyr Arg Lys Ser Val
   1 5
<210> 11
   <211> 10
   <212> PRT
   <213> PEPTIDES SYNTHETIQUES
<400> 11
   Asp Leu Gln Val Asn Ser Leu Gln Thr Val
   1 5 10
<210> 12
   <211> 10
   <212> PRT
   <213> PEPTIDES SYNTHETIQUES
<420> 12
<210> 13
   <211> 10
   <212> PRT .
   <213> PEPTIDES SYNTHETIQUES
<400> 13
   Phe Leu Pro Trp His Arg Leu Phe Leu Leu
   1 5 10
<210> 14
   <211> 9
   <212> PRT
   <213> PEPTIDES SYNTHETIQUES
<400> 14
   Met Leu Leu Ala Val Leu Tyr Cys Leu
   1 5
<210> 15
   <211> 9
   <212> PRT
   <213> PEPTIDES SYNTHETIQUES
<400> 15
   Lys Thr Trp Gly Gln Tyr Trp Gln Val
   1 5
<210> 16
   <211> 10
   <212> PRT
   <213> PEPTIDES SYNTHETIQUES
<400> 16
   Val Leu Tyr Arg Tyr Gly Ser Phe Ser Val
   1 5 10
<210> 17
   <211> 8
   <212> PRT
   <213> PEPTIDES SYNTHETIQUES
<400> 17
   Thr Asp Gln Val Pro Phe Ser Val
   1 5
<210> 18
   <211> 10
   <212> PRT
   <213> PEPTIDES SYNTHETIQUES
<400> 18
   Ser Leu Ala Asp Thr Asn Ser Leu Ala Val
   1 5 10
<210> 19
   <211> 10
   <212> PRT
   **<213> PEPTIDES SYNTHETIQUES**
<400> 19
   Ala Met Leu Gly Thr His Thr Met Glu Val
   1 5 10
<210> 20
   <211> 9
   <212> PRT
   <213> PEPTIDES SYNTHETIQUES
<400> 20
   Met Leu Gly Thr His Thr Met Glu Val
   1 5
<210> 21
   <211> 10
   <212> PRT
   <213> PEPTIDES SYNTHETIQUES
<400> 21
   Leu Leu Asp Gly Thr Ala Thr Leu Arg Leu
   1 5 10
<210> 22
   <211> 9
   <212> PRT
   <213> PEPTIDES SYNTHETIQUES
<400> 22
   Ala Ala Gly Ile Gly Ile Leu Thr Val
   1 5
<210> 23
   <211> 9
   <212> PRT
   <213> PEPTIDES SYNTHETIQUES
<400> 23
   Ile Leu Thr Val Ile Leu Gly Val Leu
   1 5
<210> 24
   <211> 9
   <212> PRT
   <213> PEPTIDES SYNTHETIQUES
<400> 24
   Cys Leu Thr Ser Thr Val Gln Leu Val
   1 5
<210> 25
   <211> 9
   <212> PRT
   <213> PEPTIDES SYNTHETIQUES
<400> 25
   His Leu Tyr Gln Gly Cys Gln Val Val
   1 5
<210> 26
   <211> 9
   <212> PRT
   <213> PEPTIDES SYNTHETIQUES
<400> 26
   Ile Leu Leu Val Val Val Leu Gly Val
   1 5
<210> 27
   <211> 9
   <212> PRT
   <213> PEPTIDES SYNTHETIQUES
<400> 27
   Gln Leu Met Pro Tyr Gly Cys Leu Leu
   1 5
<210> 28
   <211> 9
   <212> PRT
   <213> PEPTIDES SYNTHETIQUES
<400> 28
   Lys Ile Phe Gly Ser Leu Ala Phe Leu
   1 5
<210> 29
   <211> 9
   <212> PRT
   <213> PEPTIDES SYNTHETIQUES
<400> 29
   Val Leu Val Lys Ser Pro Asn His Val
   1 5
<210> 30
   <211> 9
   <212> PRT
   <213> PEPTIDES SYNTHETIQUES
<400> 30
   Ala Leu Cys Arg Trp Gly Leu Leu Leu
   1 5
<210> 31
   <211> 10
   <212> PRT
   <213> PEPTIDES SYNTHETIQUES
<400> 31
   Val Met Ala Gly Val Gly Ser Pro Tyr Val
   1 5 10
<210> 32
   <211> 9
   <212> PRT
   <213> PEPTIDES SYNTHETIQUES
<400> 32
   Glu Leu Val Ser Glu Phe Ser Arg Met
   1 5
<210> 33
   <211> 9
   <212> PRT
   <213> PEPTIDES SYNTHETIQUES
<400> 33
   Asn Leu Gln Ser Leu Thr Asn Leu Leu
   1 5
<210> 34
   <211> 10
   <212> PRT
   <213> PEPTIDES SYNTHETIQUES
<400> 34
   Leu Leu Gly Cys Ala Ala Asn Trp Ile Leu
   1 5 10
<210> 35
   <211> 9
   <212> PRT
   <213> PEPTIDES SYNTHETIQUES
<400> 35
   Leu Leu Asp Asp Glu Ala Gly Pro Leu
   1 5
<210> 36
   <211> 9
   <212> PRT
   <213> PEPTIDES SYNTHETIQUES
<400> 36
   Phe Leu Leu Ser Leu Gly Ile His Leu
   1 5
<210> 37
   <211> 9
   <212> PRT
   <213> PEPTIDES SYNTHETIQUES
<400> 37
   Pro Leu Glu Glu Glu Leu Pro Arg Leu
   1 5
<210> 38
   <211> 9
   <212> PRT
   <213> PEPTIDES SYNTHETIQUES
<400> 38
   Gly Ile Leu Gly Phe Val Phe Thr Leu
   1 5
<210> 39
   <211> 9
   <212> PRT
   <213> PEPTIDES SYNTHETIQUES
<400> 39
   Ser Leu Tyr Asn Thr Val Ala Thr Leu
   1 5
<210> 40
   <211> 9
   <212> PRT
   <213> PEPTIDES SYNTHETIQUES
<400> 40
   Ile Leu Lys Glu Pro Val His Gly Val
   1 5
<210> 41
   <211> 9
   <212> PRT
   <213> PEPTIDES SYNTHETIQUES
<400> 41
   Thr Leu Asn Ala Trp Val Lys Val Val
   1 5
<210> 42
   <211> 9
   <212> PRT
   <213> PEPTIDES SYNTHETIQUES
<400> 42
   Ala Leu Val Glu Ile Cys Thr Glu Met
   1 5
<210> 43
   <211> 11
   <212> PRT
   <213> PEPTIDES SYNTHETIQUES
<400> 43
   Val Leu Asp Val Gly Asp Ala Tyr Phe Ser Val
   1 5 10
<210> 44
   <211> 9
   <212> PRT
   <213> PEPTIDES SYNTHETIQUES
<400> 44
   Val Ile Tyr Gln Tyr Met Asp Asp Leu
   1 5
<210> 45
   <211> 9
   <212> PRT
   <213> PEPTIDES SYNTHETIQUES
<400> 45
   Ile Leu Lys Glu Pro Val His Gly Val
   1 5
<210> 46
   <211> 9
   <212> PRT
   <213> PEPTIDES SYNTHETIQUES
<400> 46
   Pro Leu Val Lys Leu Trp Tyr Gln Leu
   1 5
<210> 47
   <211> 11
   <212> PRT
   <213> PEPTIDES SYNTHETIQUES
<400> 47
   Glu Ser Glu Leu Val Asn Gln Ile Ile Glu Gln
   1 5 10
<210> 48
   <211> 10
   <212> PRT
   <213> PEPTIDES SYNTHETIQUES
<400> 48
   Glu Leu Val Asn Gln Ile Ile Glu Gln Leu
   1 5 10
<210> 49
   <211> 9
   <212> PRT
   <213> PEPTIDES SYNTHETIQUES
<400> 49
   Leu Leu Trp Lys Gly Glu Gly Ala Val
   1 5
<210> 50
   <211> 9
   <212> PRT
   <213> PEPTIDES SYNTHETIQUES
<400> 50
   Lys Leu Thr Pro Leu Cys Val Ser Leu
   1 5
<210> 51
   <211> 9
   <212> PRT
   <213> PEPTIDES SYNTHETIQUES
<400> 51
   Lys Leu Thr Pro Leu Cys Val Thr Leu
   1 5
<210> 52
   <211> 9
   <212> PRT
   <213> PEPTIDES SYNTHETIQUES
<400> 52
   Arg Leu Arg Asp Leu Leu Leu Ile Val
   1 5
<210> 53
   <211> 10
   <212> PRT
   <213> PEPTIDES SYNTHETIQUES
<400> 53
   Pro Leu Thr Phe Gly Trp Cys Phe Lys Leu
   1 5 10
<210> 54
   <211> 9
   <212> PRT
   <213> PEPTIDES SYNTHETIQUES
<400> 54
   Ala Phe His His Val Ala Arg Glu Leu
   1 5
<210> 55
   <211> 9
   <212> PRT
   <213> PEPTIDES SYNTHETIQUES
<400> 55
   Ile Leu Ala Thr Phe Leu Ala Trp Leu
   1 5
<210> 56
   <211> 10
   <212> PRT
   <213> PEPTIDES SYNTHETIQUES
<400> 56
   Phe Leu Phe Trp Leu Met Asp Thr Tyr Val
   1 5 10
<210> 57
   <211> 10
   <212> PRT
   <213> PEPTIDES SYNTHETIQUES
<400> 57
   Gln Leu Leu Arg Ser Phe Phe His Phe Leu
   1 5 10
<210> 58
   <211> 9
   <212> PRT
   <213> PEPTIDES SYNTHETIQUES
<400> 58
   Ser Leu Phe Asp Phe Phe His Phe Leu
   1 5
<210> 59
   <211> 9
   <212> PRT
   <213> PEPTIDES SYNTHETIQUES
<400> 59
   Phe Leu Ser Thr Leu Val His Gly Val
   1 5
<210> 60
   <211> 9
   <212> PRT
   <213> PEPTIDES SYNTHETIQUES
<400> 60
   Ile Leu Ala Lys Phe Leu His Trp Leu
   1 5
<210> 61
   <211> 9
   <212> PRT
   <213> PEPTIDES SYNTHETIQUES
<400> 61
   Tyr Leu Phe Phe Tyr Arg Lys Ser Val
   1 5
<210> 62
   <211> 10
   <212> PRT
   <213> PEPTIDES SYNTHETIQUES
<400> 62
   Tyr Leu Gln Val Asn Ser Leu Gln Thr Val
   1 5 10
<210> 63
   <211> 13
   <212> PRT
   <213> PEPTIDES SYNTHETIQUES
<400> 63
   Thr Pro Pro Ala Tyr Arg Pro Pro Asn Ala Pro Ile Leu
   1 5 10
<210> 64
   <211> 10
   <212> PRT
   <213> PEPTIDES SYNTHETIQUES
<400> 64
   Tyr Leu Val Pro Gln Gln Gly Phe Phe Cys
   1 5 10
<210> 65
   <211> 9
   <212> PRT
   <213> PEPTIDES SYNTHETIQUES
<400> 65
   Arg Leu Leu Gln Glu Thr Glu Leu Val
   1 5
<210> 66
   <211> 9
   <212> PRT
   <213> PEPTIDES SYNTHETIQUES
<400> 66
   Tyr Leu Ile His His Asn Thr His Leu
   1 5
<210> 67
   <211> 9
   <212> PRT
   <213> PEPTIDES SYNTHETIQUES
<400> 67
   Tyr Leu Glu Glu Ile Thr Gly Tyr Leu
   1 5
<210> 68
   <211> 9
   <212> PRT
   <213> PEPTIDES SYNTHETIQUES
<400> 68
   Tyr Leu Gln Pro Glu Gln Leu Gln Val
   1 5
<210> 69
   <211> 9
   <212> PRT
   <213> PEPTIDES SYNTHETIQUES
<400> 69
   Tyr Leu Thr Ser Ile Ile Ser Ala Val
   1 5
<210> 70
   <211> 126
   <212> PRT
   <213> PEPTIDES SYNTHETIQUES
<400> 70
   Val Met Ala Gly Val Gly Ser Pro Tyr Val Gln Leu Met Pro Tyr Gly
   1 5 10 15
   Cys Leu Leu Lys Ile Phe Gly Ser Leu Ala Phe Leu Tyr Leu Val Pro
   20 25 30
   Gln Gln Gly Phe Phe Cys Cys Leu Thr Ser Thr Val Gln Leu Val His
   35 40 45
   Leu Tyr Gln Gly Cys Gln Val Val Ala Leu Cys Arg Trp Gly Leu Leu
   50 55 60
   Leu Arg Leu Leu Gln Glu Thr Glu Leu Val Tyr Leu Ile His His Asn
   65 70 75 80
   Thr His Leu Tyr Leu Glu Glu Ile Thr Gly Tyr Leu Tyr Leu Gln Pro
   85 90 95
   Glu Gln Leu Gln Val Tyr Leu Thr Ser Ile Ile Ser Ala Val Gly Lys
   100 105 110
   Pro Ile Pro Asn Pro Leu Leu Gly Leu Asp Ser Thr Ser Thr
   115 120 125

### LISTE DE SEQUENCES

<110> INSERM
   INSTITUT GUSTAVE ROUSSY
   KOSMATOPOULOS, Kostas
   TOURDOT, Sophie
   SCARDINO, Antonio
   GROSS, David, Alexandre
<120> PROCEDE DE CRIBLAGE DE PEPTIDES UTILISABLES EN IMMUNOTHERAPIE
<130> MJPcb598-40
<140> PCT/FR01/02387
   <141> 2001-07-20
<150> FR 0009591
   <151> 2000-07-21
<160> 70
<170> PatentIn Ver. 2.1
<210> 1
   <211> 9
   <212> PRT
   <213> Séquence artificielle
<220>
   <223> Description de la séquence artificielle: peptide synthétique
<400> 1
   Ser Met Ile Glu Asn Leu Glu Tyr Met
     1               5
<210> 2
   <211> 9
   <212> PRT
   <213> Séquence artificielle
<220>
   <221> VARIANT
   <222> (2)
   <223> Xaa(1) = Tyr ou Lys et Xaa(4) = Glu ou Lys
<220>
   <223> Description de la séquence artificielle: peptide synthétique
<400> 2
   Xaa Ala Ile Xaa Asn Ala Glu Ala Leu
     1               5
<210> 3
   <211> 9
   <212> PRT
   <213> Séquence artificielle
<220>
   <223> Description de la séquence artificielle: peptide synthétique
<400> 3
   Ile Val Gly Ala Glu Thr Phe Tyr Val
   1 5
<210> 4
   <211> 9
   <212> PRT
   <213> Séquence artificielle
<220>
   <223> Description de la séquence artificielle: peptide synthétique
<400> 4
   Pro Leu Thr Ser Ile Ile Ser Ala Val
   1 5
<210> 5
   <211> 9
   <212> PRT
   <213> Séquence artificielle
<220>
   <223> Description de la séquence artificielle: peptide synthétique
<400> 5
   Ala Leu Ile His His Asn Thr His Leu
   1 5
<210> 6
   <211> 9
   <212> PRT
   <213> Séquence artificielle
<220>
   <223> Description de la séquence artificielle: peptide synthétique
<400> 6
   Thr Leu Glu Glu Ile Thr Gly Tyr Leu
   1 5
<210> 7
   <211> 9
   <212> PRT
   <213> Séquence artificielle
<220>
   <223> Description de la séquence artificielle: peptide synthétique
<400> 7
   Pro Leu Gln Pro Glu Gln Leu Gln Val
   1 5
<210> 8
   <211> 9
   <212> PRT
   <213> Séquence artificielle
<220>
   <223> Description de la séquence artificielle: peptide synthétique
<400> 8
   Glu Met Met Thr Ala Cys Gln Gly Val
   1 5
<210> 9
   <211> 10
   <212> PRT
   <213> Séquence artificielle
<220>
   <223> Description de la séquence artificielle: peptide synthétique
<400> 9
   Leu Leu Asp Thr Gly Ala Asp Asp Thr Val
   1 5 10
<210> 10
   <211> 9
   <212> PRT
   <213> Séquence artificielle
<220>
   <223> Description de la séquence artificielle: peptide synthétique
<400> 10
   Arg Leu Phe Phe Tyr Arg Lys Ser Val
   1 5
<210> 11
   <211> 10
   <212> PRT
   <213> Séquence artificielle
<220>
   <223> Description de la séquence artificielle: peptide synthétique
<400> 11
   Asp Leu Gln Val Asn Ser Leu Gln Thr Val
    1               5                  10
<210> 12
   <211> 10
   <212> PRT
   <213> Séquence artificielle
<220>
   <223> Description de la séquence artificielle: peptide synthétique
<400> 12
   Lys Leu Thr Gly Asp Glu Asn Phe Thr Ile
    1               5                  10
<210> 13
   <211> 10
   <212> PRT
   <213> Séquence artificielle
<220>
   <223> Description de la séquence artificielle: peptide synthétique
<400> 13
   Phe Leu Pro Trp His Arg Leu Phe Leu Leu
    1               5                  10
<210> 14
   <211> 9
   <212> PRT
   <213> Séquence artificielle
<220>
   <223> Description de la séquence artificielle: peptide synthétique
<400> 14
   Met Leu Leu Ala Val Leu Tyr Cys Leu
    1               5
<210> 15
   <211> 9
   <212> PRT
   <213> Séquence artificielle
<220>
   <223> Description de la séquence artificielle: peptide synthétique
<400> 15
   Lys Thr Trp Gly Gln Tyr Trp Gln Val
   1 5
<210> 16
   <211> 10
   <212> PRT
   <213> Séquence artificielle
<220>
   <223> Description de la séquence artificielle: peptide synthétique
<400> 16
   Val Leu Tyr Arg Tyr Gly Ser Phe Ser Val
   1 5 10
<210> 17
   <211> 8
   <212> PRT
   <213> Séquence artificielle
<220>
   <223> Description de la séquence artificielle: peptide synthétique
<400> 17
   Thr Asp Gln Val Pro Phe Ser Val
   1 5
<210> 18
   <211> 10
   <212> PRT
   <213> Séquence artificielle
<220>
   <223> Description de la séquence artificielle: peptide synthétique
<400> 18
   Ser Leu Ala Asp Thr Asn Ser Leu Ala Val
   1 5 10
<210> 19
   <211> 10
   <212> PRT
   <213> Séquence artificielle
<220>
   <223> Description de la séquence artificielle: peptide synthétique
<400> 19
   Ala Met Leu Gly Thr His Thr Met Glu Val
   1 5 10
<210> 20
   <211> 9
   <212> PRT
   <213> Séquence artificielle
<220>
   <223> Description de la séquence artificielle: peptide synthétique
<400> 20
   Met Leu Gly Thr His Thr Met Glu Val
   1 5
<210> 21
   <211> 10
   <212> PRT
   <213> Séquence artificielle
<220>
   <223> Description de la séquence artificielle: peptide synthétique
<400> 21
   Leu Leu Asp Gly Thr Ala Thr Leu Arg Leu
   1 5 10
<210> 22
   <211> 9
   <212> PRT
   <213> Séquence artificielle
<220>
   <223> Description de la séquence artificielle: peptide synthétique
<400> 22
   Ala Ala Gly Ile Gly Ile Leu Thr Val
   1 5
<210> 23
   <211> 9
   <212> PRT
   <213> Séquence artificielle
<220>
   <223> Description de la séquence artificielle: peptide synthétique
<400> 23
   Ile Leu Thr Val Ile Leu Gly Val Leu
   1 5
<210> 24
   <211> 9
   <212> PRT
   <213> Séquence artificielle
<220>
   <223> Description de la séquence artificielle: peptide synthétique
<400> 24
   Cys Leu Thr Ser Thr Val Gln Leu Val
   1 5
<210> 25
   <211> 9
   <212> PRT
   <213> Séquence artificielle
<220>
   <223> Description de la séquence artificielle: peptide synthétique
<400> 25
   His Leu Tyr Gln Gly Cys Gln Val Val
   1 5
<210> 26
   <211> 9
   <212> PRT
   <213> Séquence artificielle
<220>
   <223> Description de la séquence artificielle: peptide synthétique
<400> 26
   Ile Leu Leu Val Val Val Leu Gly Val
   1 5
<210> 27
   <211> 9
   <212> PRT
   <213> Séquence artificielle
<220>
   <223> Description de la séquence artificielle: peptide synthétique
<400> 27
   Gln Leu Met Pro Tyr Gly Cys Leu Leu
   1 5
<210> 28
   <211> 9
   <212> PRT
   <213> Séquence artificielle
<220>
   <223> Description de la séquence artificielle: peptide synthétique
<400> 28
   Lys Ile Phe Gly Ser Leu Ala Phe Leu
   1 5
<210> 29
   <211> 9
   <212> PRT
   <213> Séquence artificielle
<220>
   <223> Description de la séquence artificielle: peptide synthétique
<400> 29
   Val Leu Val Lys Ser Pro Asn His Val
   1 5
<210> 30
   <211> 9
   <212> PRT
   <213> Séquence artificielle
<220>
   <223> Description de la séquence artificielle: peptide synthétique
<400> 30
   Ala Leu Cys Arg Trp Gly Leu Leu Leu
   1 5
<210> 31
   <211> 10
   <212> PRT
   <213> Séquence artificielle
<220>
   <223> Description de la séquence artificielle: peptide synthétique
<400> 31
   Val Met Ala Gly Val Gly Ser Pro Tyr Val
   1 5 10
<210> 32
   <211> 9
   <212> PRT
   <213> Séquence artificielle
<220>
   <223> Description de la séquence artificielle: peptide synthétique
<400> 32
   Glu Leu Val Ser Glu Phe Ser Arg Met
   1 5
<210> 33
   <211> 9
   <212> PRT
   <213> Séquence artificielle
<220>
   <223> Description de la séquence artificielle: peptide synthétique
<400> 33
   Asn Leu Gln Ser Leu Thr Asn Leu Leu
   1 5
<210> 34
   <211> 10
   <212> PRT
   <213> Séquence artificielle
<220>
   <223> Description de la séquence artificielle: peptide synthétique
<400> 34
   Leu Leu Gly Cys Ala Ala Asn Trp Ile Leu
   1 5 10
<210> 35
   <211> 9
   <212> PRT
   <213> Séquence artificielle
<220>
   <223> Description de la séquence artificielle: peptide synthétique
<400> 35
   Leu Leu Asp Asp Glu Ala Gly Pro Leu
   1 5
<210> 36
   <211> 9
   <212> PRT
   <213> Séquence artificielle
<220>
   <223> Description de la séquence artificielle: peptide synthétique
<400> 36
   Phe Leu Leu Ser Leu Gly Ile His Leu
   1 5
<210> 37
   <211> 9
   <212> PRT
   <213> Séquence artificielle
<220>
   <223> Description de la séquence artificielle: peptide synthétique
<400> 37
   Pro Leu Glu Glu Glu Leu Pro Arg Leu
   1 5
<210> 38
   <211> 9
   <212> PRT
   <213> Séquence artificielle
<220>
   <223> Description de la séquence artificielle: peptide synthétique
<400> 38
   Gly Ile Leu Gly Phe Val Phe Thr Leu
   1 5
<210> 39
   <211> 9
   <212> PRT
   <213> Séquence artificielle
<220>
   <223> Description de la séquence artificielle: peptide synthétique
<400> 39
   Ser Leu Tyr Asn Thr Val Ala Thr Leu
   1 5
<210> 40
   <211> 9
   <212> PRT
   <213> Séquence artificielle
<220>
   <223> Description de la séquence artificielle: peptide synthétique
<400> 40
   Ile Leu Lys Glu Pro Val His Gly Val
   1 5
<210> 41
   <211> 9
   <212> PRT
   <213> Séquence artificielle
<220>
   <223> Description de la séquence artificielle: peptide synthétique
<400> 41
   Thr Leu Asn Ala Trp Val Lys Val Val
   1 5
<210> 42
   <211> 9
   <212> PRT
   <213> Séquence artificielle
<220>
   <223> Description de la séquence artificielle: peptide synthétique
<400> 42
   Ala Leu Val Glu Ile Cys Thr Glu Met
   1 5
<210> 43
   <211> 11
   <212> PRT
   <213> Séquence artificielle
<220>
   <223> Description de la séquence artificielle: peptide synthétique
<400> 43
   Val Leu Asp Val Gly Asp Ala Tyr Phe Ser Val
   1 5 10
<210> 44
   <211> 9
   <212> PRT
   <213> Séquence artificielle
<220>
   <223> Description de la séquence artificielle: peptide synthétique
<400> 44
   Val Ile Tyr Gln Tyr Met Asp Asp Leu
   1 5
<210> 45
   <211> 9
   <212> PRT
   <213> Séquence artificielle
<220>
   <223> Description de la séquence artificielle: peptide synthétique
<400> 45
   Ile Leu Lys Glu Pro Val His Gly Val
   1 5
<210> 46
   <211> 9
   <212> PRT
   <213> Séquence artificielle
<220>
   <223> Description de la séquence artificielle: peptide synthétique
<400> 46
   Pro Leu Val Lys Leu Trp Tyr Gln Leu
   1 5
<210> 47
   <211> 11
   <212> PRT
   <213> Séquence artificielle
<220>
   <223> Description de la séquence artificielle: peptide synthétique
<400> 47
   Glu Ser Glu Leu Val Asn Gln Ile Ile Glu Gln
   1 5 10
<210> 48
   <211> 10
   <212> PRT
   <213> Séquence artificielle
<220>
   <223> Description de la séquence artificielle: peptide synthétique
<400> 48
   Glu Leu Val Asn Gln Ile Ile Glu Gln Leu
   1 5 10
<210> 49
   <211> 9
   <212> PRT
   <213> Séquence artificielle
<220>
   <223> Description de la séquence artificielle: peptide synthétique
<400> 49
   Leu Leu Trp Lys Gly Glu Gly Ala Val
   1 5
<210> 50
   <211> 9
   <212> PRT
   <213> Séquence artificielle
<220>
   <223> Description de la séquence artificielle: peptide synthétique
<400> 50
   Lys Leu Thr Pro Leu Cys Val Ser Leu
   1 5
<210> 51
   <211> 9
   <212> PRT
   <213> Séquence artificielle
<220>
   <223> Description de la séquence artificielle: peptide synthétique
<400> 51
   Lys Leu Thr Pro Leu Cys Val Thr Leu
   1 5
<210> 52
   <211> 9
   <212> PRT
   <213> Séquence artificielle
<220>
   <223> Description de la séquence artificielle: peptide synthétique
<400> 52
   Arg Leu Arg Asp Leu Leu Leu Ile Val
   1 5
<210> 53
   <211> 10
   <212> PRT
   <213> Séquence artificielle
<220>
   <223> Description de la séquence artificielle: peptide synthétique
<400> 53
   Pro Leu Thr Phe Gly Trp Cys Phe Lys Leu
   1 5 10
<210> 54
   <211> 9
   <212> PRT
   <213> Séquence artificielle
<220>
   <223> Description de la séquence artificielle: peptide synthétique
<400> 54
   Ala Phe His His Val Ala Arg Glu Leu
   1 5
<210> 55
   <211> 9
   <212> PRT
   <213> Séquence artificielle
<220>
   <223> Description de la séquence artificielle: peptide synthétique
<400> 55
   Ile Leu Ala Thr Phe Leu Ala Trp Leu
   1 5
<210> 56
   <211> 10
   <212> PRT
   <213> Séquence artificielle
<220>
   <223> Description de la séquence artificielle: peptide synthétique
<400> 56
   Phe Leu Phe Trp Leu Met Asp Thr Tyr Val
   1 5 10
<210> 57
   <211> 10
   <212> PRT
   <213> Séquence artificielle
<220>
   <223> Description de la séquence artificielle: peptide synthétique
<400> 57
   Gln Leu Leu Arg Ser Phe Phe His Phe Leu
   1 5 10
<210> 58
   <211> 9
   <212> PRT
   <213> Séquence artificielle
<220>
   <223> Description de la séquence artificielle: peptide synthétique
<400> 58
   Ser Leu Phe Asp Phe Phe His Phe Leu
   1 5
<210> 59
   <211> 9
   <212> PRT
   <213> Séquence artificielle
<220>
   <223> Description de la séquence artificielle: peptide synthétique
<400> 59
   Phe Leu Ser Thr Leu Val His Gly Val
   1 5
<210> 60
   <211> 9
   <212> PRT
   <213> Séquence artificielle
<220>
   <223> Description de la séquence artificielle: peptide synthétique
<400> 60
   Ile Leu Ala Lys Phe Leu His Trp Leu
   1 5
<210> 61
   <211> 9
   <212> PRT
   <213> Séquence artificielle
<220>
   <223> Description de la séquence artificielle: peptide synthétique
<400> 61
   Tyr Leu Phe Phe Tyr Arg Lys Ser Val
   1 5
<210> 62
   <211> 10
   <212> PRT
   <213> Séquence artificielle
<220>
   <223> Description de la séquence artificielle: peptide synthétique
<400> 62
   Tyr Leu Gln Val Asn Ser Leu Gln Thr Val
   1 5 10
<210> 63
   <211> 13
   <212> PRT
   <213> Séquence artificielle
<220>
   <223> Description de la séquence artificielle: peptide synthétique
<400> 63
   Thr Pro Pro Ala Tyr Arg Pro Pro Asn Ala Pro Ile Leu
   1 5 10
<210> 64
   <211> 10
   <212> PRT
   <213> Séquence artificielle
<220>
   <223> Description de la séquence artificielle: peptide synthétique
<400> 64
   Tyr Leu Val Pro Gln Gln Gly Phe Phe Cys
   1 5 10
<210> 65
   <211> 9
   <212> PRT
   <213> Séquence artificielle
<220>
   <223> Description de la séquence artificielle: peptide synthétique
<400> 65
   Arg Leu Leu Gln Glu Thr Glu Leu Val
   1 5
<210> 66
   <211> 9
   <212> PRT
   <213> Séquence artificielle
<220>
   <223> Description de la séquence artificielle: peptide synthétique
<400> 66
   Tyr Leu Ile His His Asn Thr His Leu
   1 5
<210> 67
   <211> 9
   <212> PRT
   <213> Séquence artificielle
<220>
   <223> Description de la séquence artificielle: peptide synthétique
<400> 67
   Tyr Leu Glu Glu Ile Thr Gly Tyr Leu
   1 5
<210> 68
   <211> 9
   <212> PRT
   <213> Séquence artificielle
<220>
   <223> Description de la séquence artificielle: peptide synthétique
<400> 68
   Tyr Leu Gln Pro Glu Gln Leu Gln Val
   1 5
<210> 69
   <211> 9
   <212> PRT
   <213> Séquence artificielle
<220>
   <223> Description de la séquence artificielle: peptide synthétique
<400> 69
   Tyr Leu Thr Ser Ile Ile Ser Ala Val
   1 5
<210> 70
   <211> 126
   <212> PRT
   <213> Séquence artificielle
<220>
   <223> Description de la séquence artificielle: peptide synthétique
<400> 70
   Val Met Ala Gly Val Gly Ser Pro Tyr Val Gln Leu Met Pro Tyr Gly
   1 5 10 15
   Cys Leu Leu Lys Ile Phe Gly Ser Leu Ala Phe Leu Tyr Leu Val Pro
   20 25 30
   Gln Gln Gly Phe Phe Cys Cys Leu Thr Ser Thr Val Gln Leu Val His
   35 40 45
   Leu Tyr Gln Gly Cys Gln Val Val Ala Leu Cys Arg Trp Gly Leu Leu
   50 55 60
   Leu Arg Leu Leu Gln Glu Thr Glu Leu Val Tyr Leu Ile His His Asn
   65 70 75 80
   Thr His Leu Tyr Leu Glu Glu Ile Thr Gly Tyr Leu Tyr Leu Gln Pro
   85 90 95
   Glu Gln Leu Gln Val Tyr Leu Thr Ser Ile Ile Ser Ala Val Gly Lys
   100 105 110
   Pro Ile Pro Asn Pro Leu Leu Gly Leu Asp Ser Thr Ser Thr
   115 120 125

## Description

La vaccination ou immunothérapie peptidique est une approche thérapeutique qui fait actuellement l'objet d'un grand intérêt dans le cadre de la prévention ou du traitement des pathologies virales ou cancéreuses. Son principe repose sur l'immunisation par des peptides reproduisant des épitopes T d'antigènes viraux ou tumoraux reconnus par les cellules T cytotoxiques (CTL), en général de type CD8+, qui jouent un rôle majeur dans l'élimination des cellules exprimant ces antigènes à leur surface.

On rappellera que les CTL ne reconnaissent pas les antigènes protéiques entiers, mais des fragments peptidiques de ceux-ci, comprenant généralement 8 à 10 acides aminés, présentés par les molécules du complexe majeur d'histocompatibilité de classe I (CMH I) exprimées à la surface de différentes cellules. La présentation de ces peptides résulte d'un processus complexe, dénommé « apprêtement de l'antigène », qui implique 3 étapes principales :
- la dégradation cytosolique des protéines antigéniques par un complexe multienzymatique dénommé protéasome ;
- la translocation des peptides issus de cette dégradation dans le réticulum endoplasmique (RE) par les transporteurs TAP;
- l'association de ces peptides avec les 2 chaînes du CMH I pour former des complexes stables peptide/CMH I, qui seront exportés à la surface cellulaire.

Les complexes peptide/CMH I interagissent avec les récepteurs à l'antigène (TCR) correspondants des lymphocytes T. Cette interaction induit la stimulation de ces lymphocytes, et leur division cellulaire (prolifération clonale) qui aboutit à la génération des lymphocytes effecteurs portant le même TCR, qui assureront l'élimination de l'agresseur vis-à-vis des antigènes duquel la réponse immunitaire a été induite.

Au cours du processus d'apprêtement s'opère une sélection des peptides, qui aboutit à une hiérarchie de présentation par le CMH I à la surface de la cellule. La représentation des épitopes à la surface cellulaire dépendra notamment de la stabilité de la protéine antigénique dans le cytosol, des sites et de la fréquence des coupures effectuées par le protéasome, de l'efficacité de la translocation dans le RE par les transporteurs TAP, et surtout de la capacité des peptides à se fixer aux différentes molécules de CMH I et à former des complexes peptide/CMH I stables.

Les peptides qui sont préférentiellement présentés par le CMH à l'issue du processus d'apprêtement constituent des épitopes immunodominants, qui sont les principaux participants à la réponse CTL aux antigènes natifs dont ils sont issus. En revanche les peptides qui ne sont que faiblement présentés constituent des épitopes sous-dominants/cryptiques, qui ne participent que peu ou pas à cette réponse.

Il a été proposé d'utiliser des peptides correspondant à ceux présentés par le CMH I pour induire une réponse protectrice, notamment vis-à-vis d'antigènes viraux ou tumoraux.

Il a ainsi été montré que des vaccins basés sur des peptides immunodominants, généralement sélectionnés sur la base de leur forte affinité pour les molécules de CMH I, permettaient d'assurer une protection antivirale ou antitumorale dans de nombreux modèles expérimentaux murins et plus, récemment, chez les humains [SCHULTZ et al., Proc. Natl. Acad. Sci. USA, 88, 991 (1991) : KAST et al., Proc. Natl. Acad. Sci. USA, 88, 2283, (1991) ; MARCHAND et al., Int. J. Cancer, 80, 219, (1999) ; ROSENBERG et al., Nature Med., 4, 321, (1998)].

Cependant, il a également été montré récemment que la vaccination avec les peptides immunodominants pourrait, dans certains cas, s'avérer inefficace. Ainsi, lors de l'infection chronique avec un virus dont le taux de mutation est élevé, comme HIV ou HBV, la pression de sélection imposée par la réponse CTL antivirale naturelle favorise la survie de variants ayant muté dans la séquence de leurs peptides immunodominants. Ces variants ne sont plus reconnus par des CTL spécifiques d'épitopes immunodominants [KLENERMAN et al., Nature, 369, 403, (1994) ; BERTOLETTI et al., Nature, 369, 407, (1994) ; MOSKOPHIDIS et ZINKERNAGEL, J. Virol., 69, 2187, (1995) ; BORROW et al., Nat. Med., 3, 205, (1997) ; GOULDER et al., Nat. Med., 3, 212, (1997)].

Également, dans le cas de tumeurs exprimant à des taux élevés des protéines qui sont aussi exprimées dans des tissus normaux, et qui constituent des « antigènes du soi », un phénomène de tolérance peut se développer. Cette tolérance concerne principalement les épitopes immunodominants à forte affinité pour MHC. La stimulation du répertoire CTL spécifique de ces épitopes ne parait donc pas être la meilleure voie pour obtenir une protection antitumorale efficace.

L'utilisation d'épitopes sous-dominants/cryptiques, de faible affinité pour le CMH, a donc été proposée. Dans le cas de la vaccination antivirale, ces épitopes, qui ne sont pas soumis à une pression de sélection similaire à celle des épitopes immunodominants, peuvent représenter des cibles utiles pour éliminer des virus de type sauvage ainsi que leurs variants. Dans le cas de la vaccination antitumorale, les épitopes de faible affinité ne participant que peu ou pas à l'établissement de la tolérance, le répertoire de CTL anti-tumoraux spécifiques de ces épitopes, pourrait rester disponible pour un recrutement in *vivo.*

Lors de travaux précédents, l'équipe des Inventeurs a montré [OUKKA et al., J. Immunol., 157, 3039, (1996)] qu'il était possible d'utiliser les peptides sous-dominants/cryptiques en vaccination anti-virale. Ils ont également observé que l'efficacité de protection induite par des épitopes sous-dominants/cryptiques était inférieure à celle obtenue avec la vaccination par le peptide dominant, mais qu'elle pouvait être augmentée en rendant ces peptides plus immunogènes par l'augmentation de leur affinité pour le CMH I [TOURDOT et al., J. Immunol., 159, 2391, (1997].

La stratégie habituelle pour augmenter l'immunogénicité des épitopes viraux ou tumoraux, consiste à augmenter leur affinité pour le CMH I et/ou la stabilité du complexe peptide/CMH I par des substitutions d'acides aminés. Il a en effet été observé que les peptides capables de former un complexe avec un allèle de CMH donné ont en commun la présence, à certaines positions, de résidus d'acides aminés conservés. On a ainsi défini pour chaque allèle du CMH I un motif d'ancrage spécifique, impliquant des acides aminés dénommés « résidus d'ancrage primaires ». Il a aussi été montré que des résidus situés en dehors des sites d'ancrage (résidus d'ancrage secondaires) pouvaient exercer un effet favorable ou défavorable sur l'affinité du peptide pour le CMH ; la présence de ces résidus d'ancrage secondaires permet d'expliquer qu'il existe, au sein des peptides possédant le même motif d'ancrage spécifique d'un CMH I donné, une grande variabilité dans l'affinité de fixation, et que des peptides n'ayant pas le motif d'ancrage primaire complet puissent être présentés par les molécules de CMH I et posséder pour ces molécules une forte affinité.

De nombreuses équipes sont ainsi parvenues à augmenter l'immunogénicité de peptides identifiés comme immunogènes viraux ou tumoraux potentiels, en augmentant leur affinité pour le CMH I. Par exemple, chez la souris, LIPFORD et al. [Vaccine, 13, 313, (1995)] ont montré que la substitution de D par I en position 2 du peptide sur l'épitope 50-57 de l'Ag E6.1 du papillomavirus présenté par la molécule K^{b} augmentait la stabilité des complexes formés avec la molécule K^{b}, et rendait l'épitope immunogène *in vivo,* les CTL induits reconnaissant les cellules transformées par le papillomavirus. BRISTOL et al. [J. Immunol., 160, 2433, (1998)] ont également montré que le remplacement du résidu V en position C-terminale de l'épitope 4-12 de l'oncogène Ras p21 muté, par I ou L, qui sont les acides aminés d'ancrage en position 9 spécifiques de la molécule K^{d}, permettait l'induction d'une réponse CTL spécifique chez la souris BALB/c. HUDRISIER et al. [Mol. Immunol., 32, 895, (1995)] ont identifié les résidus du peptide SMIENLEYM (SEQ ID NO: 1) qui intervenaient dans la liaison à la molécule D^{b}, et ont produit une série de peptides à forte affinité, dérivés de la séquence X¹AIX⁴NAEAL (SEQ ID NO: 2) où X¹=Y ou K et X⁴=E ou K.

Chez l'homme, POGUE et al. [Proc. Natl. Acad. Sci. USA, 92, 8166, (1995)] ont substitué des acides aminés aux différentes positions de l'épitope immunogène 767-484 de la transcriptase inverse du virus HIV-1, présenté par la molécule HLA A2.1 avec une forte affinité, et ont montré que la substitution par Y ou F du résidu en position 1 augmentait l'affinité déjà forte du peptide et sa capacité à induire des CTL à partir de PBL de donneurs séropositifs possédant l'allèle HLA A2.1. PARKHURST et al. [J. Immunol., 157, 2539, (1996)] ont réalisé des substitutions uniques aux positions 1, 2, 3, ou doubles aux positions 1 et 2, ou 2 et 3, dans les épitopes immunogènes gp100 209, gp100 280 et gp100 154 de l'Ag gp100 associé au mélanome, et ont montré que les épitopes modifiés gp100 209 2M, et gp100 280 9V avaient une affinité plus forte que l'épitope non-modifié. BAKKER et al. [Int. J. Cancer, 70, 302, (1997)], ont obtenu par substitution à une des positions d'ancrage (position 2) ou en dehors des positions d'ancrage (position 8), des variants de l'épitope gp100 154, possédant une plus forte affinité que l'épitope natif. SAROBE et al. [J. Clin. Invest., 102, 1239, (1998)] ont obtenu un variant immunogène de l'épitope C7A de la protéine core du virus HCV présenté par HLA A2.1. VALMORI et al. [J. Immunol., 160, 1750, (1998)] ont obtenu un dérivé à forte affinité de l'épitope 26-35 de l'antigène de mélanome MART-1 présenté par HLA A2.1. MICHELETTI et al. [Eur.J.Immunol. 1999, 29, 2579] ont effectué des simples ou des doubles substitutions aux positions 1 et/ou 3 du peptide CLG qui provient de la protéine LMP-2 du virus EBV. Dans sa forme native, ce peptide a une forte affinité pour la molécule du CMH et est immunogène. Les auteurs ont observé que les variants A3 et Y1-A3 issus du peptide CLG ont une plus forte affinité que le peptide natif, et stimulent une plus forte réponse immunologique.

Il apparaît donc possible d'augmenter l'immunogénicité d'épitopes sous-dominants/cryptiques, afin de les utiliser en immunothérapie. Cependant, ceci nécessite l'identification préalable de ces épitopes. Or, celle-ci demeure problématique, du fait précisément de leur faible immunogénicité.

Dans le but de remédier à ce problème, les Inventeurs ont recherché s'il était possible de définir des règles générales de substitution des acides aminés qui permettrait d'augmenter l'affinité et donc l'immunogénicité de la majorité des épitopes tumoraux présentés par le CMH (par les molécules HLA A2.1), et ce, qu'ils possèdent ou non les acides aminés d'ancrage spécifiques de cette molécule, et de façon générale, indépendamment de leur séquence d'origine.

Les Inventeurs ont ainsi constaté que la seule substitution de l'acide aminé N-terminal par un résidu tyrosine augmentait, quelle que soit la séquence du peptide natif, l'affinité de ce peptide pour les molécules HLA de classe I et notamment la molécule HLA A2.1, et la stabilité du complexe peptide/CMH I formé, et ce dans une proportion d'autant plus importante que l'affinité du peptide natif était plus faible. Ils ont en outre observé que les peptides modifiés de la sorte conservaient la spécificité antigénique des peptides naturels, et devenaient immunogènes, et capables e recruter in vivo un répertoire CTL spécifique du peptide natif correspondant.

La présente invention a pour objet un procédé d'identification d'épitopes sous-dominants/cryptiques présentés par la molécule HLA A2.1 de classe I, caractérisé en ce qu'il comprend au moins les étapes suivantes :
a) le choix, à partir de la séquence d'une protéine vis-à-vis de laquelle on souhaite induire une réponse cytotoxique T, d'au moins une séquence peptidique de 8 à 11 acides aminés qui possède tout ou partie du motif d'ancrage primaire de la molécule HLA A2.1, et telle que le complexe de ce peptide avec la molécule HLA A2.1 possède une DC50 inférieure à 2 heures :
b) la préparation, pour chaque séquence sélectionnée, d'un peptide variant dérivé de ladite séquence par substitution de l'acide aminé N-terminal par un résidu tyrosine ;
c) la détermination de l'immunogénicité de chaque peptide variant obtenu à l'étape b) par la sélection, parmi ceux-ci, de chaque peptide immunogène générant une réponse CTL spécifique vis-à-vis de cellules cibles exprimant la protéine dont est issue la séquence peptidique choisie à l'étape a), et l'identification de la séquence peptidique dont dérive ledit peptide immunogène.

Dans le cadre de l'exposé de la présente invention, on entend par « peptide immunogène » un peptide capable d'induire une réponse CTL spécifique et par « peptide non-immunogène » un peptide incapable d'induire une réponse CTL spécifique.

Le choix des séquences peptidiques susceptibles de constituer des épitopes présentés par une molécule HLA de classe I, HLA A2.1 peut s'effectuer, de manière classique, par l'analyse de la séquence peptidique de la protéine choisie, afin de sélectionner les peptides possédant tout ou partie du motif d'ancrage primaire d'une molécule HLA A2.1 (L en position 2 et/ou V/L en position C-terminale). On éliminera, bien entendu, les peptides connus comme étant immunogènes. Eventuellement, on pourra effectuer une sélection complémentaire des peptides dont la capacité de liaison potentielle à HLA A2.1, prédite par analyse informatique, apparaît la plus faible. Des algorithmes utilisables dans ce but sont connus en eux-mêmes ; à titre d'exemple, on citera ceux décrits par PARKER et al. [J. Immunol., 152, 163, (1994)].

Cette analyse pourra avantageusement être complétée par la détermination expérimentale de l'affinité de liaison du peptide pour HLA A2.1, et de la stabilité du complexe peptide/HLA 12.1. Des peptides non-immunogènes présentent le plus souvent une faible affinité pour HLA de classe I, et/ou forment avec celui-ci un complexe peu stable. Des méthodes pour déterminer l'affinité du peptide pour HLA A2.1, et la stabilité du complexe formé sont connues en elles-mêmes. On citera par exemple celle décrite par FIRAT et al. [Eur. J. Immunol., 29, 3112, (1999)].

L'affinité d'un peptide pour HLA de classe I, notamment HLA A2.1 est le plus souvent définie par rapport à celle d'un peptide de référence, sous forme d'affinité relative. L'affinité relative est définie comme le rapport de la concentration dudit peptide permettant la formation d'une certaine quantité de complexe peptide/HLA de classe I, à la concentration du peptide de référence permettant la formation, dans les mêmes conditions, de la même quantité de complexe peptide/HLA de classe I. Dans ce cas, plus l'affinité relative est importante, plus l'affinité de liaison du peptide pour HLA de classe I sera faible.

A titre d'exemple, pour un complexe peptide/HLA A2.1, en prenant comme peptide de référence, le peptide de séquence IVGAETFYV (SEQ ID NO: 3), plus de 84 % des peptides non-immunogènes auront fréquemment une affinité relative supérieure à 10.

La stabilité du complexe peptide/HLA A2.1 est souvent définie par la DC50, qui représente le temps nécessaire à la dissociation de 50% de complexes formés. Généralement, ce temps est inférieur à 2 heures pour les peptides non-immunogènes [VAN DER BURG et al., J. Immunol., 156, 3308 (1996)].

Ainsi, si un peptide présenté par HLA A2.1, possède une affinité relative (par rapport à HIVpol 589) supérieure à 10, et une DC50 inférieure à 2 heures, il sera très probablement non-immunogène.

L'immunogénicité des peptides retenus à l'étape c) pourra être facilement vérifiée, par exemple par des méthodes classiques de détermination de la capacité de ce peptide à générer, *in vivo, ex vivo*, ou *in vitro* une réponse CTL spécifique vis-à-vis de cellules cibles exprimant la protéine dont il est issu.

Une fois ce choix effectué, les peptides variants, dérivés des séquences retenues par substitution de l'acide aminé N-terminal par un résidu tyrosine, peuvent être très facilement préparés, notamment par synthèse peptidique, selon des techniques classiques bien connues en elles-même de l'homme du métier.

Avant la détection de l'immunogénicité de ces peptides variants, effectuée à l'étape c) du procédé conforme à l'invention, ledit procédé comprend une présélection desdits peptides variants par la détermination de l'affinité relative du peptide pour une molécule HLA A2.1, et/ou de la stabilité du complexe peptide/HLA de classe I formé, et la sélection des peptides variants présentant une affinité relative pour HLA A2.1 inférieure à celle des peptides natifs dont ils sont dérivés, et/ou une DC50 supérieure à 2 heures.

Chaque peptide répondant à ces critères peut ensuite être testé, pour rechercher s'il peut induire un répertoire CTL spécifique du peptide natif non-immunogène dont il dérive, et capable notamment d'entraîner la lyse de cellules-cibles exprimant la protéine native dont ce peptide non-immunogène est issu.

Si ce test est positif, on peut considérer que ce peptide natif non-immunogène représente un épitope sous-dominant/cryptique, présenté par HLA A2.1, dudit antigène.

La mise en oeuvre du procédé ci-dessus a par exemple permis aux Inventeurs d'identifier de nouveaux épitopes sous-dominants/cryptiques présentés par HLA A2.1 de l'antigène tumoral HER-2/neu, de la sous-unité catalytique de la télomérase (TERT) et du virus HIV-1.

Ces épitopes sous-dominants/cryptiques sont les suivants :
Pour HER-2/neu :
   le peptide HER-2/neu 650 : PLTSIISAV (SEQ ID NO: 4)
   le peptide HER-2/neu 466 : ALIHHNTHL (SEQ ID NO: 5)
   le peptide HER-2/neu 402 : TLEEITGYL (SEQ ID NO: 6)
   le peptide HER-2/neu 391 : PLQPEQLQV (SEQ ID NO: 7)
Pour HIV-1 :
   le peptide gagp24-212 : EMMTACQGV (SEQ ID NO: 8)
   le peptide pol79 : LLDTGADDTV (SEQ ID NO: 9)
Pour la sous-unité catalytique de la télomérase (TERT) :
   le peptide mhp 572 : RLFFYRKSV (SEQ ID NO: 10)
   le peptide mhp 988 : DLQVNSLQTV (SEQ ID NO: 11)

La présente spécification a également pour objet des épitopes peptidiques immunogènes dérivés d'épitopes sous-dominants/cryptiques pouvant être identifiés par la mise en oeuvre du procédé conforme à l'invention, et notamment des épitopes sous-dominants/cryptiques de HER/neu, du virus HIV-1, et de la sous-unité catalytique de la télomérase (TERT) mentionnés ci-dessus.

Un épitope peptidique immunogène conforme à l'invention peut être obtenu par différentes modifications de la séquence peptidique de l'épitope sous-dominant/cryptique dont il dérive. Très avantageusement, ces modifications comprennent au moins la substitution de l'acide aminé N-terminal par un résidu tyrosine. Il peut s'agir notamment d'un peptide variant sélectionné à l'issue de l'étape c) du procédé conforme à l'invention, ou éventuellement de tout dérivé de celui-ci comprenant d'autres modifications de séquence permettant d'accroître son immunogénicité.

La présente invention a également pour objet des compositions comprenant au moins un épitope peptidique immunogène conforme à la revendication 4.

Les épitopes peptidiques immunogènes conformes à l'invention sont utilisables dans tous les traitements d'immunothérapie pour lesquels il est souhaitable d'induire une réponse CTL dirigée contre des épitopes sous-dominants/cryptiques, et notamment dans le cadre de la thérapie antivirale ou anti-tumorale.

Avantageusement, il s'agit de compositions multiépitopiques, capables de générer une réponse CTL polyspécifique, et qui dans ce but comprennent également un ou plusieurs autre (s) épitope(s) immunogène(s). Il peut s'agir d'un ou plusieurs autre(s) épitope(s) sous-dominant(s)/cryptique(s) et/ou d'un ou plusieurs épitope(s) immunodominant(s). Ces épitopes peuvent être issus du même antigène, ou de deux ou plusieurs antigènes différents.

Avantageusement, des compositions multiépitopiques conformes à l'invention peuvent également comprendre au moins un épitope présenté par une molécule du CMH II, et capable d'induire une réponse T auxiliaire. Elles peuvent comprendre en outre, pour être plus largement utilisables sur une population dont les individus portent des allèles HLA différents, un ou plusieurs épitopes présentés par des molécules du CMH I autres que HLA A2.

Selon un mode de réalisation préféré d'une composition conforme à l'invention, elle comprend au moins un polypeptide chimérique comprenant une ou plusieurs copies d'un épitope peptidique immunogène conforme à l'invention. Dans le cas d'une composition multiépitopique, ledit polypeptide chimérique comprend en outre une ou plusieurs copies d'au moins un autre épitope immunogène.

Un tel polypeptide chimérique peut être facilement obtenu par des méthodes connues en elles-mêmes, et notamment par les techniques classiques de l'ADN recombinant.

La présente invention a également pour objet une molécule d'acide nucléique codant un polypeptide chimérique conforme à l'invention.

La présente invention a également pour objet l'utilisation d'un épitope peptidique immunogène de la revendication 4, d'une composition, ou d'une molécule d'acide nucléique conforme à l'invention pour l'obtention d'un médicament, et notamment d'un médicament destiné à l'immunothérapie antivirale ou antitumorale.

La présente invention englobe également les médicaments comprenant, en tant que principe actif, au moins un épitope peptidique immunogène de la revendication 4, une composition, ou une molécule d'acide nucléique conforme à l'invention.

Selon un mode de réalisation préféré de la présente invention, lesdits médicaments sont des vaccins.

Des médicaments conformes à l'invention peuvent comprendre en outre les excipients usuels, ainsi que des adjuvants habituellement utilisés en immunothérapie, et permettant par exemple de favoriser l'administration du principe actif, de le stabiliser, d'augmenter son immunogénicité, etc.

La présente invention sera mieux comprise à l'aide du complément de description qui va suivre, qui se réfère à des exemples non-limitatifs d'identification de nouveaux peptides et de nouveaux antigènes potentiellement utilisables en immunothérapie, par la mise en oeuvre du procédé conforme à l'invention.

### EXEMPLE 1 : RELATION ENTRE L'AFFINITE, LA STABILITE DES COMPLEXES HLA A2.1/PEPTIDE ET L'IMMUNOGENICITE DES PEPTIDES VIRAUX ET TUMORAUX.

Trente-cinq peptides provenant d'antigènes viraux (HBV, HIV, Flu) et de tumeurs (HER-2/neu, mélanome gp100, Tyrosinase, Mart-1) ont été testés pour leur capacité à fixer et à stabiliser la molécule HLA A2.1 : ces peptides sont représentés dans le Tableau I ci-après.

**Tableau I**

| Peptides | Séquences |
|---|---|
| Tyrosinase 224 | KLTGDENFTI (SEQ ID NO: 12) |
| Tyrosinase 207 | FLPWHRLFLL (SEQ ID NO: 13) |
| Tyrosinase 1* | MLLAVLYCL (SEQ ID NO: 14) |
| gp100 154* | KTWGQYWQV (SEQ ID NO: 15) |
| gp100 476* | VLYRYGSFSV (SEQ ID NO: 16) |
| gp100 209* | TDQVPFSV (SEQ ID NO: 17) |
| gp100 570* | SLADTNSLAV (SEQ ID NO: 18) |
| gp100 177* | AMLGTHTMEV (SEQ ID NO: 19) |
| gp100 178 | MLGTHTMEV (SEQ ID NO: 20) |
| gp100 457* | LLDGTATLRL (SEQ ID NO: 21) |
| mart-1 27* | AAGIGILTV (SEQ ID NO: 22) |
| mart-1 32* | ILTVILGVL (SEQ ID NO: 23) |
| HER-2/neu 789* | CLTSTVQLV (SEQ ID NO: 24) |
| HER-2/neu 48 | HLYQGCQW (SEQ ID NO: 25) |
| HER-2/neu 650 | PLTSIISAV (SEQ ID NO: 4) |
| HER-2/neu 466 | ALIHHNTHL (SEQ ID NO: 5) |
| HER-2/neu 402 | TLEEITGYL (SEQ ID NO: 6) |
| HER-2/neu 661 | ILLVVVLGV (SEQ ID NO: 26) |
| HER-2/neu 799* | QLMPYGCLL (SEQ ID NO: 27) |
| HER-2/neu 369* | KIFGSLAFL (SEQ ID NO: 28) |
| HER-2/neu 851* | VLVKSPNHV (SEQ ID NO: 29) |
| HER-2/neu 5* | ALCRWGLLL (SEQ ID NO: 30) |
| HER-2/neu 391 | PLQPEQLQV (SEQ ID NO: 7) |
| HER-2/neu 773* | VMAGVGSPYV (SEQ ID NO: 31) |
| HER-2/neu 971* | ELVSEFSRM (SEQ ID NO: 32) |
| HER-2/neu 1023* | YLVPQQGFFC (SEQ ID NO: 64) |
| HER-2/neu 689* | RLLQETELV (SEQ ID NO: 65) |
| HBVpol 985 | NLQSLTNLL (SEQ ID NO: 33) |
| HBVpol 765 | LLGCAANWIL (SEQ ID NO: 34) |
| HBVpol 28 | LLDDEAGPL (SEQ ID NO: 35) |
| HBV pol 575* | FLLSLGIHL (SEQ ID NO: 36) |
| H BVpol 594 | PLEEELPRL (SEQ ID NO: 37) |
| FluM 58* | GILGFVFTL (SEQ ID NO: 38) |
| HIVgag 76* | SLYNTVATL (SEQ ID NO: 39) |
| HIVrt 309* | ILKEPVHGV (SEQ ID NO: 40) |

Vingt-deux de ces peptides (signalés par un astérisque dans le Tableau I) sont déjà connus en tant qu'épitopes, présentés par HLA A2.1 et générant une réponse cytotoxique CTL chez l'homme ; les 13 autres peptides qui n'ont pas encore été décrits en tant qu'épitopes, ont été choisis en fonction de deux critères : la présence de motifs d'ancrage primaires de HLA A2.1 (L en position 2 et V/L en position C-terminale) et un score de liaison faible, selon une évaluation effectuée par le programme BIMAS [PARKER et al., J. Immunol., 152, 163, (1994)].

L'affinité pour HLA A2.1 et la stabilité du complexe peptide/HLA A2.1 ont été évaluées par cytométrie de flux et l'immunogénicité a été évaluée par génération de CTL sur des souris transgéniques HHD [PASCOLO et al., J. Exp. Med., 185, 2043, (1997)].

Les protocoles utilisés sont les suivants [FIRAT et al., Eur. J. Immunol., 29, 3112, (1999)] :

### Affinité :

Des cellules T2 [FIRAT et al., Eur. J. Immunol., 29, 3112, (1999)] (3x10⁵ cellules/ml) humaines, qui sont déficientes en transporteurs TAP, sont incubées à 37°C pendant 16 heures avec diverses concentrations de chaque peptide à tester dans du milieu RPMI 1640 sans sérum, supplémenté avec 100 ng/ml de β2-microglobuline humaine. Ensuite, elles sont lavées deux fois, et marquées avec l'anticorps monoclonal BB7.2 [PARHAM et al., Hum. Immunol., 3, 4, 277-299, (1981)] qui est spécifique de la molécule HLA A2.1, puis avec un anticorps de chèvre anti-Ig de souris, couplé à l'isothiocyanate de fluorescéine (FITC).

Les cellules sont ensuite analysées en cytométrie de flux. Pour chaque concentration de peptide, la fluorescence spécifique de HLA A2.1 est calculée en tant que % de la fluorescence obtenue avec 100 µM d'un peptide de référence (HIVpol 589 ; IVGAETFYV (SEQ ID NO: 3)). L'affinité relative (RA) est définie comme le rapport de la concentration de chaque peptide induisant 20% de la fluorescence obtenue avec 100 µM du peptide de référence, à la concentration du peptide de référence induisant 20% de la fluorescence obtenue avec 100 µM dudit peptide de référence. Plus l'affinité relative est faible, et plus fortement le peptide se lie à HLA A2.1. La RA moyenne pour chaque peptide est déterminée à partir d'au moins trois expériences indépendantes. Dans toutes les expériences, 20% de la fluorescence maximale ont été obtenus pour 1 à 3 µM du peptide de référence.

### Stabilité :

Des cellules T2 (10⁶/ml) sont incubées pendant une nuit à 37°C avec 100 µM de chaque peptide à tester dans du milieu RPMI 1640 sans sérum, supplémenté avec 100 ng/ml de β2-microglobuline humaine. Ensuite, elles sont lavées à quatre reprises pour éliminer les peptides libres, incubées avec du Brefeldin A (SIGMA ; 10 µg/ml) pendant une heure pour prévenir l'expression à leur surface des molécules HLA A2.1 nouvellement synthétisées, lavées et incubées à 37°C pendant 0, 2, 4, 6 ou 8 heures. Pour chaque temps d'incubation, les cellules sont ensuite marquées, comme indiqué ci-dessus, avec l'anticorps BB7.2, et analysées en cytométrie de flux pour évaluer la quantité de complexe peptide/HLA A2.1 présent à leur surface. Cette quantité est évaluée par la formule : (fluorescence moyenne des cellules T2 préincubées avec le peptide - fluorescence moyenne des cellules T2 traitées dans des conditions similaires en l'absence de peptide). Le DC50 (complexe de dissociation : DC) est défini comme étant le temps requis pour la perte de 50% des complexes HLA A2.1/peptide stabilisés à t=0.

### Immunogénicité :

Les souris HHD utilisées sont β2m-/-, D^{b}-/- et expriment une monochaîne HLA A2.1 composée des domaines α1 et α2 de HLA A2.1 et des domaines α3 et intracellulaire de D^{b} reliée par son N-terminal au C-terminal de β2-m humain par un peptide de 15 acides aminés.

Les souris reçoivent une injection sous-cutanée à la base de la queue avec 100 µg de chaque peptide à tester émulsifié dans de l'adjuvant incomplet de Freund, en présence de 140 µg d'un épitope auxiliaire T dérivé de l'antigène « core » de HBV (128-140, séquence TPPAYRPPNAPIL (SEQ ID NO: 63)).

Après 11 jours, des cellules spléniques prélevées sur les souris (5x10⁷ cellules dans 10 ml) sont stimulées *in vitro* avec le peptide à tester (10 µM). Le 6ème jour de culture, les populations qui répondent sont testées pour déterminer une cytotoxicité spécifique. Dans certains cas, les cellules qui répondent sont restimulées *in vitro* à des intervalles d'une semaine avec 2x10⁶ cellules spléniques HHD irradiées (3000 rads) et 10 µM de peptide en présence de 20 UI/ml d'IL2 recombinante.

Des cellules RMA-HHD et RMAS-HHD, sont utilisées comme cibles pour étudier la cytotoxicité. Ces cellules sont respectivement obtenues par transfection de cellules RMA murines et de leurs cellules RMAS variantes déficientes en TAP avec la construction HHD comme décrit par PASCOLO et al. [J. Exp. Med., 185, 2043, (1997)]. Elles sont infectées par des virus exprimant les différents antigènes dont sont issus les peptides à tester.

Les virus utilisés sont les suivants : le virus recombinant de la vaccine exprimant VIHgag VVTG1144 (vac-VIHgag) décrit par JOHNSON [J. Immunol., 147, 1512, (1991)] ; le virus recombinant de la vaccine exprimant HER-2/neu VT39 (vac-neu) (Therion Biologics) ; le virus de la vaccine vac-gp100, décrit par YANG [J. Immunol., 164, 4204, (2000)] ; un virus de la vaccine de type sauvage (vac-WT) ; et le virus flu PR8 de l'influenza décrit par VIRELIZIER [J. Immunol . 115, 2, 434-439, (1975)]. Pour les infections virales, les cellules RMA-HHD sont incubées pendant 16 heures avec les virus de la vaccine (10 PFU/cellule), recombinants ou sauvage, ou avec le virus flu PR8 (50 HAU) pendant 2 heures.

Les cellules-cibles sont marquées avec 150 µCi de ⁵¹Cr pendant 90 minutes, puis lavées trois fois et étalées dans des plaques de 96 puits à fond rond (10⁴ cellules/puits dans 100 µl de RPMI 1640 + 3% de sérum de veau foetal).

Des cellules RMA-HHD ou RMAS-HHD non infectées sont chargées avec 1 µM de peptide à tester, à 37°C pendant 90 minutes.

Ensuite, 100 µl des cellules effectrices à différentes concentrations, sont ajoutés dans les puits et les plaques sont incubées à 37°C pendant 4 heures. Après incubation, 100 µl de surnageant sont collectés et la radioactivité est mesurée dans un compteur γ.

Le pourcentage de lyse spécifique est calculé par la formule : [(libération de ⁵¹Cr expérimentale-libération de ⁵¹Cr spontanée) / (libération de ⁵¹Cr maximale-libération de ⁵¹Cr spontanée)] x 100. Dans toutes les expériences, la libération spontanée est inférieure à 20% de la libération maximale induite par HCl 3N.

En fonction de leur capacité à fixer et à stabiliser la molécule HLA A2.1, et de leur immunogénicité, les 34 peptides ont été classés en trois groupes différents ; les résultats de ce classement sont représentés dans le Tableau II.

**Tableau II**

| Peptides | RA | DC50 (heures) | Répondeurs*/Souris totales |
|---|---|---|---|
| **Groupe I** | | | |
| HIV gag 76 | 1,0 | >8 | 7/10 |
| Flu M58 | 0,2 | >8 | 4/6 |
| HBV pol 575 | 2,5 | >8 | 6/8 |
| HBV pol 765 | 2,0 | 4 | ND |
| Mart-1 27 | 2,2 | 2-4 | 4/5 |
| gp100 177 | 0,5 | >6 | 3/5 |
| gp 100178 | 0,3 | 6-8 | 4/6 |
| gp 100 154 | 2,3 | 6-8 | 7/9 |
| gp 100 570 | 1,0 | 4-6 | 6/9 |
| gp100 209 | 1,3 | 4 | 4/6 |
| gp100 476 | 10,0 | 6 | 8/10 |
| gp 100 457 | 1,6 | 2-4 | 4/6 |
| HER-2/neu 799 | 1, 0 | 6-8 | 3/4 |
| HER-2/neu 369 | 2,3 | 4 | 12/13 |
| HER-2/neu 789 | 1,6 | 6-8 | 4/6 |
| HER-2/neu 48 | 1,7 | >8 | 5/6 |
| HER-2/neu 773 | 1,7 | 6 | 2/3 |
| HER-2/neu 5 | 2,3 | >8 | 5/6 |
| HER-2/neu 689 | 2 | 4 | 5/6 |

| **Groupe II** | | | |
|---|---|---|---|
| Tyrosinase 1 | >60,0 | 2-4 | 4/23 |
| Mart-1 32 | 21,1 | 4 | 0/10 |
| HER-2/neu 851 | 24,0 | 4 | 1/12 |
| HER-2/neu 661 | >60,0 | 2-4 | 0/6 |
| HER-2/neu 1023 | 19,6 | 4 | 5/10 |

| **Groupe III** | | | |
|---|---|---|---|
| HBV pol 28 | 5,3 | <2 | 0/6 |
| HBV pol 594 | 4,2 | <2 | 0/6 |
| HBV pol 985 | 43,3 | <2 | 0/6 |
| Tyrosinase 224 | >50,0 | <2 | 0/6 |
| Tyrosinase 207 | >50,0 | 2 | 0/6 |
| HER-2/neu 650 | 1,4 | <2 | 0/6 |
| HER-2/neu 466 | 4,8 | 2 | 0/6 |
| HER-2/neu 402 | 19,0 | <2 | 0/6 |
| HER-2/neu 391 | >70,0 | 2 | 0/6 |
| HER-2/neu 971 | >70,0 | 2 | 0/6 |

| | | | |
|---|---|---|---|
| * On considère que les souris répondent lorsqu'une cytotoxicité spécifique contre des cribles pulsés par un peptide est supérieure à 15% de la toxicité vis-à-vis des cibles non-chargées. | | | |

Le premier groupe est constitué de 19 peptides ayant une RA ≤ 10 et un DC50 > 2 heures. Ils correspondent à des épitopes d'antigènes de virus ou de tumeurs (à l'exception de HBVpol 765 et HER-2/neu 48) et déclenchent une réponse CTL chez un pourcentage élevé (60 à 92%) des souris HHD.

Le deuxième groupe de cinq peptides avec une RA > 10 et un DC50 > 2 heures comprend trois épitopes connus, et un épitope potentiel (HER-2/neu 661). Deux d'entre eux sont non-immunogènes (Mart-1 32, HER-2/neu 661), tandis que HER-2/neu 851 et tyrosinase 1 induisent une réponse chez un faible pourcentage de souris HHD (8% et 17%, respectivement).

Dix peptides avec un DC50 < 2 heures et une RA variable appartiennent au troisième groupe. Ils ne correspondent pas à des épitopes connus (à l'exception de HER-2/neu 971) et ils sont non-immunogènes chez des souris HHD, même s'ils ont une RA élevée, comme HBVpol 28, HBVpol 594, HER-2/neu 650 et HER-2/neu 466.

Les conclusions suivantes peuvent être tirées de ces résultats : (i) des motifs d'ancrage secondaires influencent fortement la liaison HLA A2.1 puisque des peptides ayant les résidus d'ancrage primaires HLA A2.1 optima présentent un très large spectre d'affinités, (ii) l'affinité de liaison n'est pas toujours en corrélation avec l'aptitude à stabiliser la molécule HLA A2.1. Les peptides Mart-1 32 et HER-2/neu 851 sont de faibles liants mais ils forment des complexes peptide/HLA A2.1 stables. Au contraire, les peptides HBVpol 28, HBVpol 594, HER-2/neu 650 et HER-2/neu 466 sont des liants puissants mais ils forment des complexes instables avec la molécule HLA A2.1, (iii) l'immunogénicité des peptides dépend principalement de leur capacité à stabiliser la molécule HLA A2.1. Des peptides ayant un DC50 < 2 heures ne sont jamais immunogènes même s'ils ont une forte affinité de liaison. Cependant, une stabilité de HLA A2.1 induite par un peptide n'est pas suffisante pour assurer une immunogénicité. En fait, des peptides ayant un DC50 > 2 heures peuvent être non immunogènes (Mart-1 32 et HER-2/neu 661) ou très faiblement immunogènes (Tyrosinase 1 et HER-2/neu 851) s'ils présentent une faible affinité de liaison.

Il apparaît donc qu'il est nécessaire d'améliorer à la fois l'affinité de liaison et l'aptitude à stabiliser la molécule HLA A2.1 pour que des peptides génèrent une forte réponse CTL.

### EXEMPLE 2 : EFFET DU REMPLACEMENT DU RESIDU EN POSITION P1 PAR UNE TYROSINE SUR L'AFFINITE ET LA STABILITE DU COMPLEXE HLA A2.1/PEPTIDE.

Des variants de 33 peptides décrits dans l'exemple 1, résultant de la substitution du 1^{er} acide aminé N-terminal par une tyrosine (substitution P1Y), ont été synthétisés. Ces variants ont été testés pour leur affinité pour la molécule HLA A2.1 et leur capacité à stabiliser le complexe formé.

Les résultats sont illustrés par le Tableau III.

**Tableau III**

| Peptide | RA WT/RA Y1 | DC50 Y1-DC50 WT |
|---|---|---|
| HIV gag 76 | ↑ 3,0 | = 0 |
| Flu M58 | ↑ 2,4 | = 0 |
| H BV pol 575 | ↑ 2,6 | = 0 |
| HBV pol 765 | ↑ 40,4 | ↑ 4 |
| Mart-127 | ↑ 2 | = 0 |
| gp100177 | = 0,6 | ↑ 2 |
| gp100178 | = 0,8 | ↑ 2 |
| gp100 154 | = 0,8 | ↓ 2 |
| gp100 570 | ↑ 3,4 | ↑ >2 |
| gp100209 | = 1,7 | ↑ 2 |
| gp100476 | ↑4,2 | ↑ 2 |
| gp 100 457 | ↑2.3 | ↑ >2 |
| HER-2/neu 369 | ↑3,9 | ↑ >2 |
| HER-2/neu 799 | ↑ 3,9 | ↑ >2 |
| HER-2/neu 789 | ↑2,1 | = 0 |
| HER-2/neu 48 | ↑ 3,0 | = 0 |
| HER-2/neu 773 | ↑ 2,0 | ↑ 2 |
| HER-2/neu 5 | = 1,1 | = 0 |
| HER-2/neu 689 | ↑ 3,1 | ↑ >2 |
| Tyrosinase 1 | ↑ >3,7 | ↑ >2 |
| Mart-1 32 | ↑ 16,2 | ↑ 2 |
| HER-2/neu 851 | ↑ 3,0 | ↑ 2 |
| HER-2/neu 661 | ↑ >1,5 | ↑ 2 |
| HBV pol 28 | ↑ 2,3 | ↑ >2 |
| HBV pol 594 | ↑ 14,8 | ↑ >6 |
| H BV pol 985 | ↑ 13,7 | ↑ >6 |
| Tyrosinase 224 | ↑ >5,1 | ↑ >2 |
| Tyrosinase 207 | ↑ >6,4 | ↑ >4 |
| HER-2/neu 650 | ↑ 6,0 | ↑ >4 |
| HER-2/neu 466 | ↑ 3,3 | t >4 |
| HER-2/neu 402 | ↑ 5,2 | ↑ >2 |
| HER-2/neu 391 | ↑ 55,5 | ↑ >6 |
| HER-2/neu 971 | ↑ 11,6 | ↑ 2 |

Ces résultats montrent que la substitution P1Y augmente la liaison de tous les peptides de faible affinité, et favorise la stabilisation de HLA A2.1 pour tous les peptides stabilisants faibles (groupes II et III dans le Tableau II). L'augmentation d'affinité, mesurée par le rapport entre les RA du peptide modifié et du peptide naturel, est au minimum de 1,5 et va jusqu'à 55,5, tandis que l'augmentation de la stabilisation de HLA A2.1, mesurée par la différence entre les DC50 du peptide modifié et du peptide naturel est au minimum de 2 heures et va jusqu'à 6 heures. Le RA de tous les peptides modifiés, à l'exception d'un d'entre eux (HER-2/neu 661Y1) est inférieur à 10, et leur DC50 est > 4 heures. Pour les peptides Tyrosinase 1 et HER-2/neu 661, la modification P1Y ne génère pas de peptides ayant une très forte affinité. Ceci est dû à la présence dans ces peptides de résidus d'ancrage secondaires P3-P8/9 défavorables pour la liaison de HLA A2.1.

L'effet de la substitution P1Y n'est pas limité aux peptides de faible affinité mais est aussi observé avec la majorité des peptides de forte affinité (groupe I du le Tableau II). Deux seulement des dix-neuf peptides de forte affinité n'ont amélioré ni leur affinité de liaison, ni leur capacité de stabilisation (gp100 154 et HER-2/neu 5). Il faut noter qu'une augmentation d'affinité est indépendante de la nature du résidu en position 1 du peptide natif et qu'elle est observée même si le résidu substitué n'est pas un résidu défavorable à la liaison avec HLA A2.1. Quatre seulement des 19 peptides ayant un rapport RA du peptide naturel/RA de peptide modifié supérieur à 3 ont un résidu défavorable en P1 (P pour HER-2/neu 391, HER-2/neu 650 et HBVpol 594, E pour HER-2/neu 971). L'augmentation d'affinité de ces quatre peptides est toutefois très importante : elle est comprise entre 6 et 55,5.

Cependant, l'affinité est augmentée même lorsque Y substitue un autre résidu favorable comme F (HBVpol 575 et Tyrosinase 207).

Ces résultats démontrent qu'une substitution P1Y augmente la liaison à HLA A2.1 et la capacité de stabilisation du complexe formé pour presque tous les peptides liés à HLA A2.1. Cet effet est bien plus prononcé pour les peptides non-immunogènes de faible affinité pour HLA A2.1.

### EXEMPLE 3 : RECONNAISSANCE CROISEE DES PEPTIDES NATURELS ET DE LEURS VARIANTS P1Y PAR DES CTL SPECIFIQUES.

L'augmentation de l'affinité pour HLA A2.1 est la première condition pour rendre immunogènes des peptides de faible affinité. Il est cependant également nécessaire que leur conformation dans le complexe avec HLA A2.1 ne soit pas modifiée, et que leur spécificité antigénique soit conservée. Si tel est le cas, des CTL générés chez des souris HHD vaccinées par le peptide natif doivent reconnaître celui-ci et son variant P1Y avec la même efficacité. De plus, les variants P1Y doivent être capables de recruter *in vivo* le répertoire des CTL spécifiques du peptide naturel.

La reconnaissance des variants P1Y par des CTL spécifiques de peptide naturel a d'abord été étudiée. Des CTL induits chez des souris HHD sensibilisées avec les peptides HIVgag 76, HBVpol 575, gp100 154, gp100 457, gp100 476, gp100 570, gp100 177 ou HER-2/neu 369 ont été testés pour leur capacité à tuer des cibles RMAS-HHD chargées avec soit les peptides naturels (WT), soit les variants P1Y correspondants (P1Y).

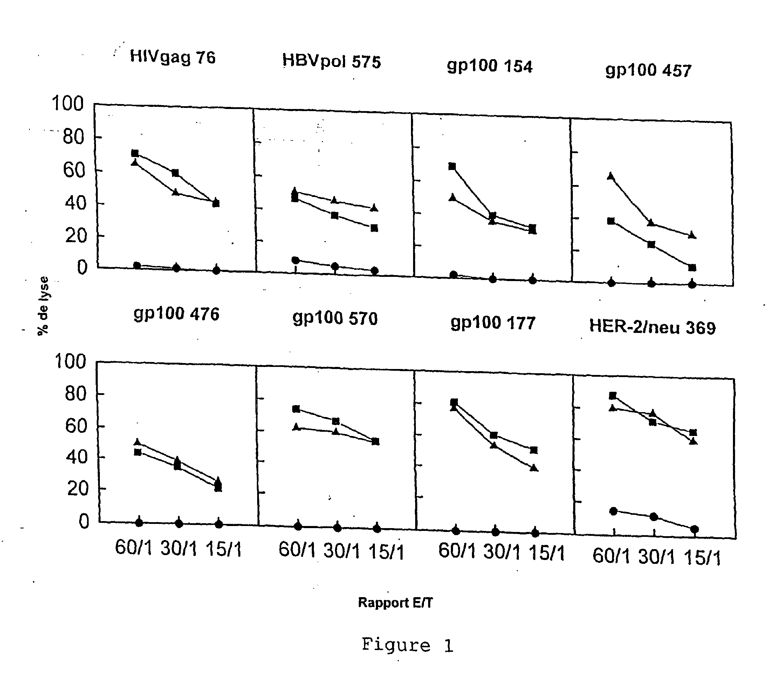

La Figure 1 illustre les résultats (% de lyse en fonction du rapport cellules effectrices/cellules cibles) obtenus avec huit peptides différents.
Cellules RMAS-HHD chargées avec le peptide naturel : ■
Cellules RMAS-HHD chargées avec le peptide P1Y : ▲
Cellules RMAS-HHD non-chargées : ●.

Ces résultats montrent que des CTL induits chez des souris HHD sensibilisées avec les peptides HIVgag 76, HBVpol 575, gp100 154, gp100 457, gp100 476, gp100 570, gp100 177 et HER-2/neu 369 tuent avec la même efficacité les cellules RMAS-HHD chargées avec le peptide naturel ou avec son variant P1Y.

Les variants P1Y sont aussi capables de recruter les CTL spécifiques du peptide naturel in vivo. Des cellules spléniques de souris HHD sensibilisées avec les variants P1Y HIVgag 76Y1, HBVpol 575Y1, gp100 154Y1, gp100 457Y1; gp100 476Y1, gp100 570Y1, gp100 177Y1 et HER-2/neu 369Y1, sont testées pour leur capacité à tuer des cibles RMAS-HHD chargées avec soit les variants P1Y (P1Y), soit avec les peptides de type sauvage (WT).

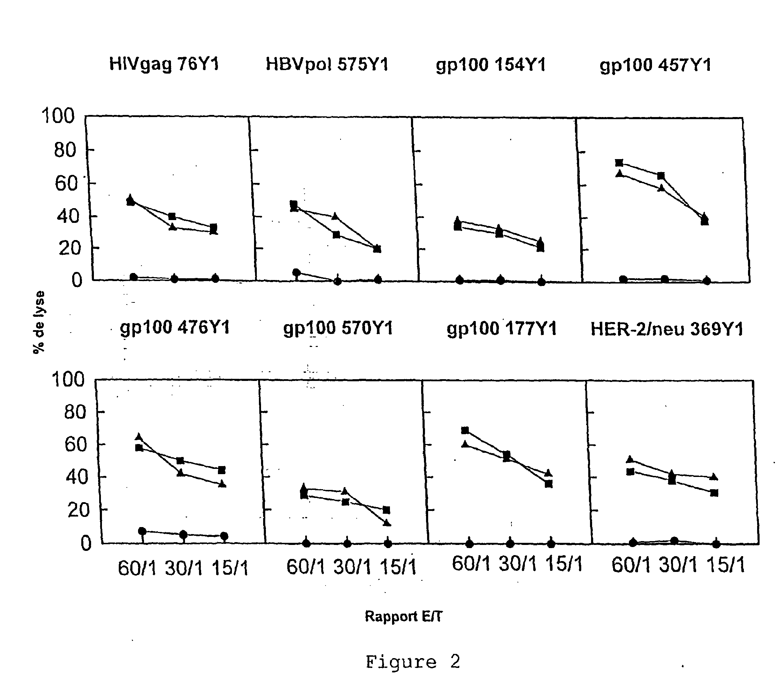

Les résultats (% de lyse en fonction du rapport cellules effectrices/cellules cibles) sont illustrés par la Figure 2.
Cellules RMAS-HHD chargées avec le peptide naturel : ■
Cellules RMAS-HHD chargées avec le peptide P1Y : ▲
Cellules RMAS-HHD non-chargées : ●.

Ces résultats montrent que les variants P1Y génèrent des CTL qui tuent les cibles RMAS-HHD chargées avec le peptide variant, ou avec le peptide naturel correspondant.

En outre, tous ces peptides variants à l'exception de gp100 154Y1, induisent des CTL chez un pourcentage plus élevé de souris HHD que ne font les peptides naturels correspondants. Trois à huit souris HHD ont été testées pour chaque peptide et une réponse CTL a été induite chez 100% des souris sensibilisées par HIVgag 76Y1,^{.} HBVpol 575Y1, gp100 476Y1, gp100 570Y1 et HER-2/neu 369Y1 et chez 75% des souris sensibilisées par gp100 457Y1, gp100 177Y1 et gp100 154Y1.

De plus, les CTL induits par les variants P1Y reconnaissent ces variants et les peptides naturels correspondants avec des avidités comparables.

Les CTL générés chez des souris sensibilisées avec les variants P1Y HER-2/neu 369Y1, HIVgag 76Y1 et gp100 154Y1 ont été testés pour leur aptitude à tuer des cibles RMAS-HHD chargées avec différentes concentrations du peptide variant P1Y, ou du peptide sauvage correspondant.

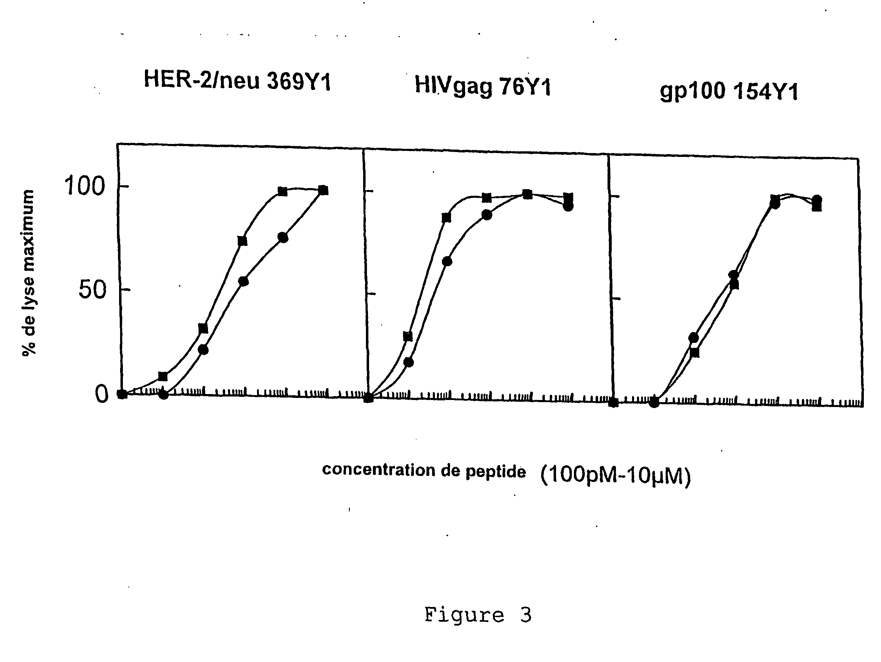

Les résultats (% de lyse en fonction du rapport cellules effectrices/cellules cibles) sont illustrés par la Figure 3.
Cellules RMAS-HHD chargées avec le peptide naturel: ●
Cellules RMAS-HHD chargées avec le peptide P1Y : ■.

Ces résultats montrent que les CTL induits par les variants P1Y donnent une cytolyse égale à la moitié de la cytolyse maximale pour des quantités similaires de variant P1Y et de peptide naturel.

Pour envisager une utilisation des variants P1Y en immunothérapie, il est en outre nécessaire que les CTL induits par ces variants reconnaissent des épitopes apprêtés naturellement de l'antigène dont ils sont dérivés.

Des CTL générés chez des souris sensibilisées par HIVgag 76Y1, HER-2/neu 369Y1, HER-2/neu 5Y1, gp100 476Y1 et fluM 58Y1 sont testés pour déterminer leur aptitude à tuer des cellules RMA-HHD infectées par les virus recombinants vac-HIVgag, vac-neu, vac-gp100 ou flu PR8 et exprimant de façon endogène les antigènes viraux correspondants, ou infectées avec le virus sauvage vac-WT.

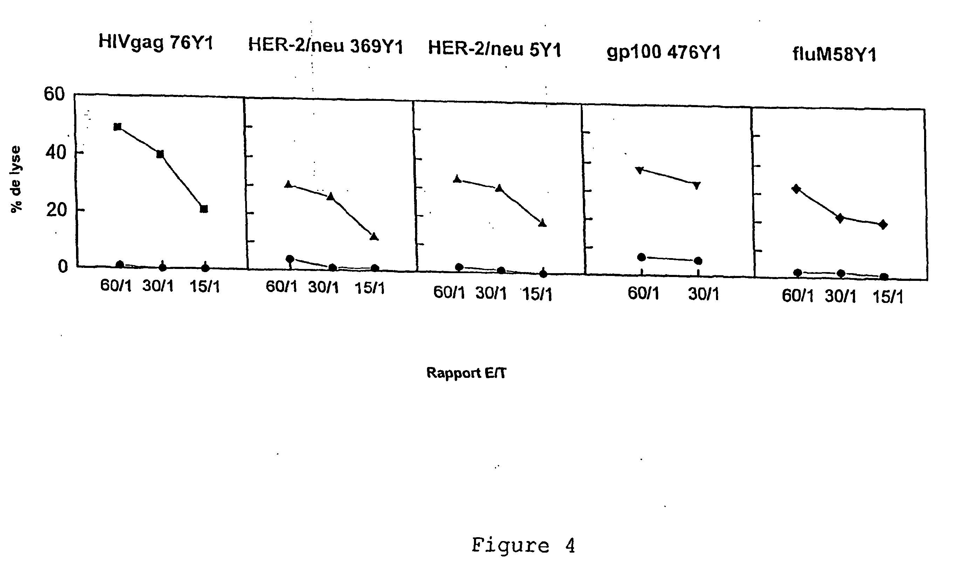

Les résultats (% de lyse en fonction du rapport cellules effectrices/cellules cibles) sont illustrés par la Figure 4 :
Cellules RMA-HHD infectées avec vac-WT (●) ;
Cellules RMA-HHD infectées avec vac-HIVgag (■) ;
Cellules RMA-HHD infectées avec vac-neu (▲) ;
Cellules RMA-HHD infectées avec vac-gp100 (▼) ;
Cellules RMA-HHD infectées avec flu PR8 (◆).

Ces résultats montrent que des CTL spécifiques des variants P1Y reconnaissent l'épitope maturé naturellement puisqu'ils tuent des cibles infectées avec le virus correspondant mais non les cibles infectées par vac-wt.

Il apparaît donc que la substitution P1Y satisfait aux deux critères qui sont nécessaires à l'induction d'une réponse CTL contre un quelconque peptide de faible affinité pour HLA A2.1, quelle que soit sa séquence. D'abord, elle augmente l'affinité de liaison et la capacité de stabilisation des peptides liés à HLA A2.1 et en second lieu, elle n'interfère pas avec l'interaction peptide/TCR et ne modifie donc pas leur spécificité antigénique.

### EXEMPLE 4 : RESTAURATION DE L'IMMUNOGENICITE DE PEPTIDES NON IMMUNOGENES DE FAIBLE AFFINITE POUR HLA A2.1 PAR UNE SUBSTITUTION P1Y.

Des souris ont été vaccinées avec les variants P1Y des peptides HER-2/neu 402, HER-2/neu 466, HER-2/neu 650, HER-2/neu 391, Tyrosinase 207, HBVpol 594, HBVpol 28 et HBVpol 985. Onze jours plus tard, leurs cellules spléniques sont restimulées *in vitro* avec le peptide naturel correspondant, et les CTL générés sont testés contre des cellules-cibles RMAS-HHD non-chargées ou chargées avec le peptide naturel.

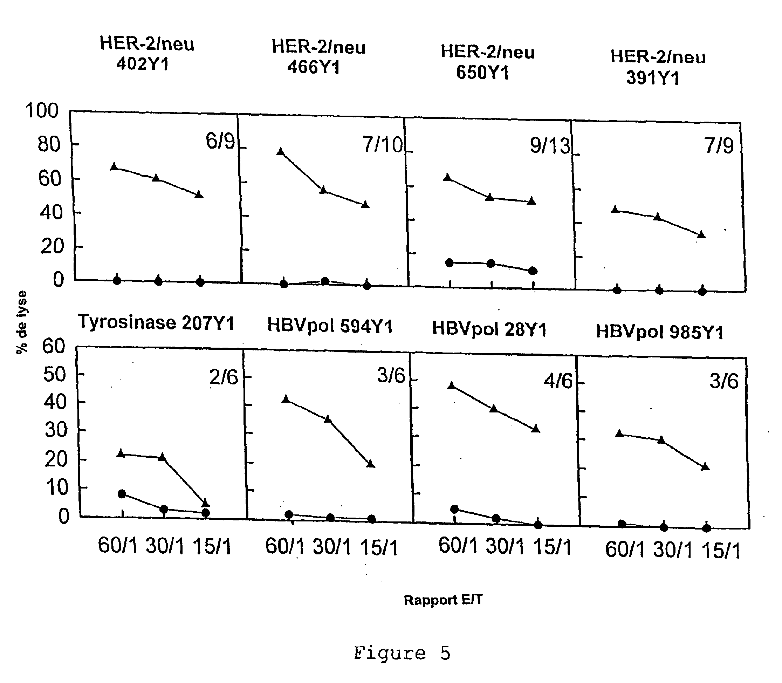

Les résultats (% de lyse en fonction du rapport cellules effectrices/cellules cibles) sont illustrés par la Figure 5 :
Cellules RMAS-HHD chargées avec le peptide naturel : ▲
Cellules RMAS-HHD non-chargées : ●.

Pour chaque peptide, le nombre de souris qui répondent est indiqué.

Ces résultats montrent que les souris HHD sensibilisées avec des variants P1Y génèrent des CTL spécifiques du peptide naturel. En outre, le pourcentage des souris qui fournissent une réponse est relativement élevé : il est compris entre 33 et 77%.

Ces données démontrent qu'une substitution P1Y constitue une stratégie générale pour augmenter l'immunogénicité de peptides non-immunogènes de faible affinité pour HLA A2.1.

### EXEMPLE 5 : IDENTIFICATION D'EPITOPES SOUS-DOMINANTS/CRYPTIQUES A L'AIDE DE VARIANTS P1Y.

La possibilité d'induire une réponse CTL contre des peptides de faible affinité pour HLA A2.1 permet d'identifier des épitopes sous-dominants/cryptiques viraux ou tumoraux, utiles pour une immunothérapie spécifique.

Cette possibilité est illustrée ci-après par l'exemple de 3 antigènes différents : l'antigène tumoral HER-2/neu, le virus HIV-1 et la sous-unité catalytique de la télomérase (hTERT).

### Epitopes HER-2/neu :

Le peptide HER-2/neu 650 ne participe pas à la réponse CTL spécifique de HER-2/neu développée chez des patients portant une tumeur HER-2/neu+. 2 hypothèses sont possibles : ou bien il s'agit d'un épitope qui n'est pas naturellement maturé par des cellules tumorales exprimant HER-2/neu, ou bien il s'agit d'un épitope sous-dominant/cryptique.

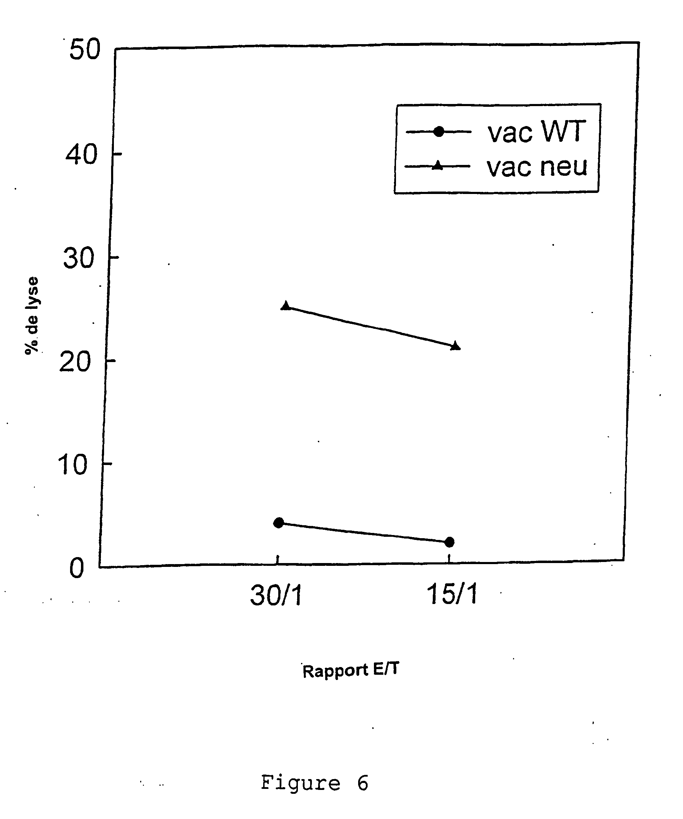

Comme décrit à l'Exemple 1 ci-dessus, le peptide HER-2/neu 650 forme des complexes HLA A2.1/peptide instables et n'est donc pas immunogène.
1) Pour étudier si le peptide HER-2/neu 650 correspond à un épitope sous-dominant/cryptique, les CTL générés chez des souris sensibilisées par le variant HER-2/neu 650Y1 sont testés pour leur capacité à tuer des cellules RMA-HHD infectées par vac-neu.
   Les résultats (% de lyse en fonction du rapport cellules effectrices/cellules cibles) sont illustrés par la Figure 6 :
   Cellules RMA-HHD infectées avec vac-WT (●) ;
   Cellules RMA-HHD infectées avec vac-neu (▲).

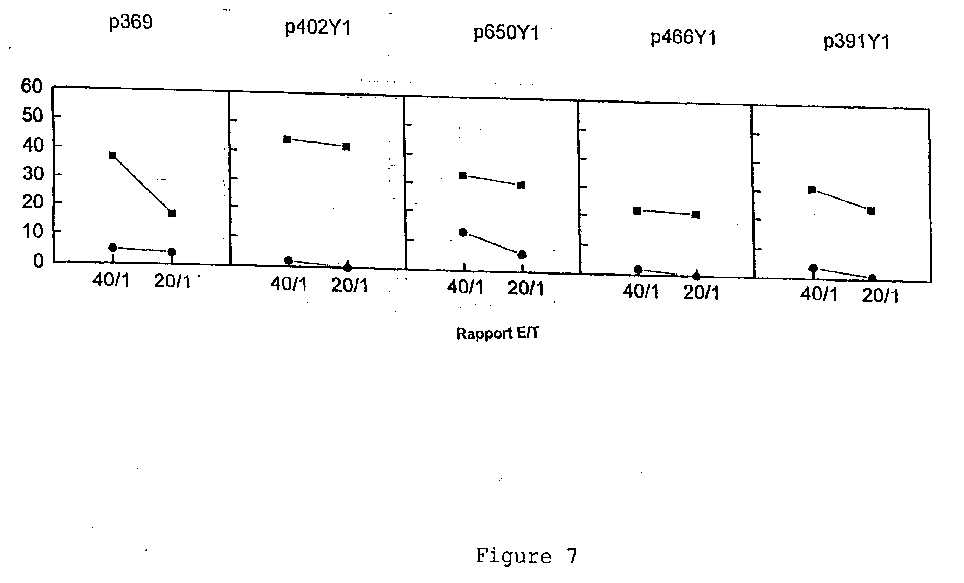

   Ces résultats montrent que des CTL spécifiques de HER-2/neu 650 tuent des cellules cibles infectées par vac-neu, mais non des cibles infectées par vac-WT, ce qui démontre que le peptide HER-2/neu 650 est un épitope sous-dominant/cryptique de HER-2/neu.
   Les variants P1Y des peptides HER-2/neu 466, HER-2/neu 402, HER-2/neu 661 et HER-2/neu 391 ont été testés de la même manière ; à l'exception du variant P1Y de HER-2/neu 661, les résultats sont similaires à ceux observés pour HER-2/neu 650.
   Les peptides HER-2/neu 466, HER-2/neu 402 et HER-2/neu 391 constituent donc également des épitopes sous-dominant/cryptiques de HER-2/neu.
   En ce qui concerne HER-2/neu 661, les résultats obtenus confirment ceux relatifs à la faible augmentation de l'affinité décrits à l'exemple 2 ci-dessus. Des modifications supplémentaires devront être effectuées pour envisager l'utilisation de ce peptide en immunothérapie.
2) L'immunogénicité des variants HER-2/neu 466Y1, HER-2/neu 402Y1, HER-2/neu 650Y1, et HER-2/neu 391Y1 conformes à l'invention, par rapport à un peptide immunodominant a également été évaluée sur des cellules humaines, et comparée à celle du peptide immunodominant HER-2/neu 369.
   Des cellules mononucléées du sang périphérique (PMBC) ont été obtenues à partir de donneurs sains HLA A2.1, et resuspendues dans 2 ml de milieu de culture RPMI 1640 supplémenté avec 10 nM de glutamine, 250 unités/ml de pénicilline-streptomycine, et, 10% de sérum humain AB inactivé par la chaleur, et incubés à 37°C pendant 2 heures. Les cellules non-adhérentes sont collectées, et les cellules adhérentes restantes sont chargées avec le peptide à tester (5µM) à 37°C pendant 90 minutes, et irradiées à 3000 rads ; elles sont ensuite lavées pour éliminer le peptide libre.
   3x10⁶ cellules non-adhérentes sont ajoutées à un volume final de 2 ml de milieu de culture supplémenté avec 50 UI/ml d'IL-2 recombinante. Au septième jour, elles sont récoltées, lavées, et suspendues dans du milieu de culture, et restimulées avec 5x10⁶ cellules autologues adhérentes préalablement chargées avec le peptide à tester comme décrit ci-dessus. Le lendemain, 150 UI/ml d'IL-2 recombinante sont ajoutées. Le neuvième ou le dixième jour, les cultures sont complémentées avec 300 UI/ml d' IL-2 recombinante. Lorsque nécessaire, le milieu est changé en prélevant 1 ml de surnageant de culture et en le remplaçant par 1 ml de milieu de culture contenant 300 UI/ml d'IL-2 recombinante. Cette procédure est répétée au moins 3 fois à intervalles d'une semaine.
   Des cellules T2 chargées avec le peptide natif (1 µM de peptide, 37°C , 90 minutes) correspondant au peptide P1Y à tester sont utilisées comme cibles pour étudier la cytotoxicité.
   Les résultats (% de lyse en fonction du rapport cellules effectrices/cellules cibles) pour chacun des peptides testés sont illustrés par la Figure 7 :
   Cellules T2 chargées avec le peptide naturel : ■
   Cellules T2 non-chargées : ●.

   Ces résultats montrent que la substitution P1Y augmente également l'immunogénicité vis-à-vis de cellules humaines.
3) Pour vérifier si les peptides P1Y induisaient des CTL humains spécifiques d'épitopes tumoraux naturellement apprêtés, des CTL générés, comme décrit ci-dessus, à partir de PBMC humains, provenant de 3 donneurs différents, stimulés par HER-2/neu 369, ou par les peptides HER-2/neu 466 Y1, HER-2/neu 402, HER-2/neu 650 Y1, et HER-2/neu 391 Y1 ont également été testés pour leur capacité à tuer des cellules de tumeurs humaines HLA A2.1+, exprimant en quantité plus ou moins importante l'épitope HER-2/neu.

Les cellules tumorales utilisées comme cellules-cibles sont les suivantes :
- 4 lignées cellulaires HLA A2.1+/ HER-2/neu+ : MC F-7 [ZAKS, Cancer Res., 58, 4902, (1998)] ; PUB/N (lignée tumorale humaine de cancer du poumon « pas à petites cellules ») ; HCT-116 [BROSSART, Cancer Res., 58, 732, (1998)] ; LAW (lignée tumorale humaine de cancer rénal) ;
- 2 lignées cellulaires HLA A2.1+/ HER-2/neu- : ZR75.1 [OSBORNE et al., Cancer Res., 39, 2422-2428, (1979)] ; SUP/M2 [MORGAN et al., Blood, 73, 8, 2155-2164, (1989)] ;
- à titre de témoin, la lignée K562, sensible à la lyse par les cellules NK.

L'expression de l'antigène tumoral HER-2/neu par ces cellules, évaluée par immunofluorescence à l'aide d'un anticorps monoclonal anti-HER-2/neu, est illustrée par le Tableau IV ci-après.

**Tableau IV**

| Lignée cellulaire | HER-2/neu (FI) |
|---|---|
| ZR75.1 | 0,12 |
| SUP/M2 | 0,11 |
| MCF-7 | 1,94 |
| HCT-116 | 1,23 |
| PUBIN | 1,33 |
| LAW | 0,35 |

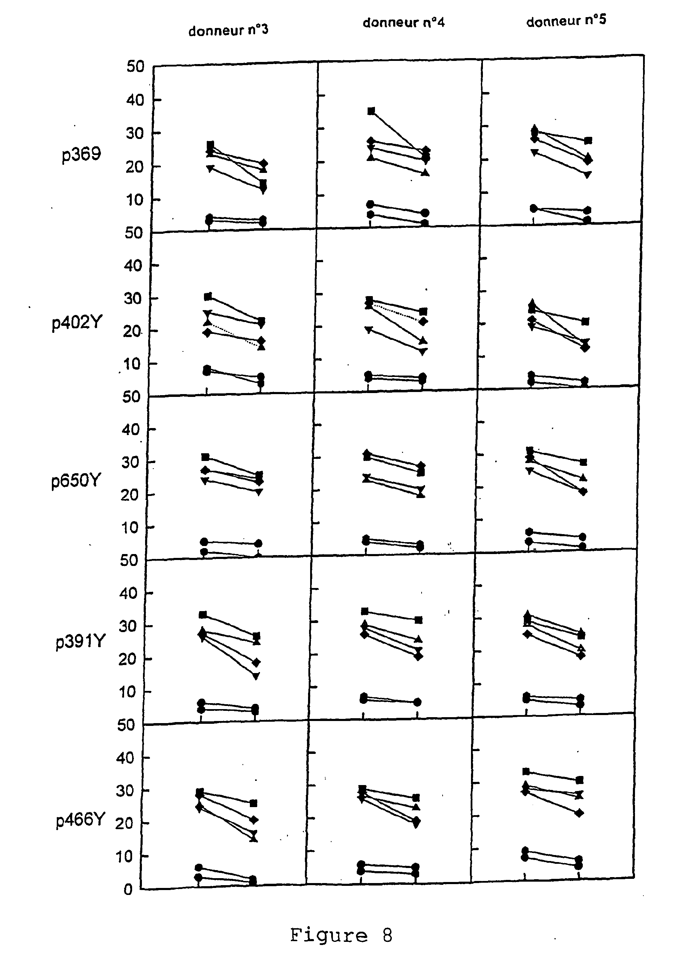

Les résultats de ces essais de cytotoxicité (% de lyse en fonction du rapport cellules effectrices/cellules cibles ; rapports E/T : 40/1 ; 20/1) pour chacun des peptides testés sont illustrés par la Figure 8 :
Cellules ZR75.1 :●
Cellules SUP/M2 : 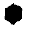
Cellules MCF-7 : ■
Cellules HCT-116 : ▼
Cellules PUB/N : ▲
Cellules LAW : ◆.

Aucune cytotoxicité vis-à-vis des cellules K562 n'est observée.

Ces résultats montrent que les CTL obtenus à partir des PMBC de 3 donneurs différents, stimulés par les peptides variants P1Y, lysent les cellules HER-2/neu+ MCF-7, PUB/N, HCT-116, et LAW, mais pas les cellules HER-2/neu-ZR75.1 et SUP/M2.

Il est à noter que la lyse est aussi efficace dans le cas des cellules LAW, qui n'expriment que faiblement l'antigène HER-2/neu que dans celui des cellules MCF-7, PUB/N, et HCT-116, qui l'expriment à un niveau élevé. Ceci montre que la présentation des épitopes de faible affinité ne nécessite pas l'expression de l'antigène à un niveau élevé.

### Epitopes de HIV 1 :

La substitution P1Y a été utilisée pour identifier de nouveaux épitopes sous-dominants/cryptiques de HIV 1.

17 peptides de Gag, Pol, Env et Nef de HIV, ont été choisis à partir de la séquence polypeptidique complète de HIV 1. La liste de ces peptides est représentée dans le Tableau V ci-après.

**Tableau V**

| Protéine d'origine* | | Peptides | Fréquence parmi les isolats de HIV 1 (%)** |
|---|---|---|---|
| GAG | p17 (77-85) | SLYNTVATL (SEO ID NO: 39) (S9L) | 39.6 |
| | p24 (19-27) | TLNAVVVKVV (SEQ ID NO: 41) (T9V) | 62.5 |
| | p24 (212-221) | EMMTACQGV (SEQ ID NO: 8) (E9V) | 95.8 |
| POL | (79-88) | LLDTGADDTV (SEQ ID NO: 9) (L10V) | 96.8 |
| | (188-196) | ALVEICTEM (SEQ ID NO: 42) (A9M) | 46.2 |
| | (263-273) | VLDVGDAYFSV (SEQ ID NO: 43) (V11V) | 84.9 |
| | (334-342) | VIYQYMDDL (SEQ ID NO: 44) (V9L) | 84.9 |
| | (464-472) | ILKEPVHGV (SEQ ID NO: 45) (I9V) | 68.8 |
| | (576-584) | PLVKLWYQL (SEQ ID NO: 46) (P9L) | 87.1 |
| | (669-679 | ESELVNQIIEQ (SEQ ID NO: 47) (E11Q) | 33.3 |
| | (671-680) | ELVNQIIEQL (SEQ ID NO: 48) (E10L) | 33.3 |
| | (956-964) | LLWKGEGAV (SEQ ID NO: 49) (L9V) | 98.9 |
| ENV | gp120 (120-128) | KLTPLCVSL (SEQ ID NO: 50) (K9L) | 8.9 |
| | gp120 (120-128) | KLTPLCVTL (SEQ ID NO: 51) (K9L/T) | 79.4 |
| | gp41 (260-268) | RLRDLLLIV (SEQ ID NO: 52) (R9V) | 0.3 |
| NEF | (134-143) | PLTFGWCFKL (SEQ ID NO: 53) (P10L) | 0.1 |
| | (188-196) | AFHHVAREL (SEQ ID NO: 54) (A9L) | |

| | | | |
|---|---|---|---|
| * La numérotation des acides aminés est basée sur la séquence du clone HIV 1 WEAU 1.60 Numéro d'accès Genbank U21135). Cette référence est simplement donnée pour indiquer la localisation des peptides de ce tableau par rapport aux protéines virales ; la séquence de ces peptides n'est pas toujours totalement identique à celle des peptides correspondants du clone WEAU. *^{,}*La fréquence a été calculée à partir des isolats disponibles sur la base de données « HIV Molecular Immunology Database ». | | | |

L'affinité relative de ces peptides pour HLA A2.1 (peptide de référence I9V), et la stabilité du complexe peptide/ HLA A2.1 ont été déterminées comme décrit dans l'exemple 1 ci-dessus, pour le peptide natif, et pour son variant résultant de la substitution de l'acide aminé N-terminal par une tyrosine.

Les résultats sont illustrés dans le Tableau VI ci-dessous :

**Tableau VI.**

| Peptides épitopiques CD8 | | Peptides natifs | | Peptides P1Y | |
|---|---|---|---|---|---|
| | | RA | DC | RA | DC |
| GAG | S9L | 2,2 | >6h | 5 | >6h |
| | T9V | 5,5 | 3,5h | 5,5 | >6h |
| | E9V | 21 | <2h | 1,7 | >6h |
| POL | L10V | 10 | <2h | 1,5 | 4h |
| | A9M | 5 | 2h | 1,75 | >6h |
| | V11V | 4,5 | 2h | 2,5 | 3,5h |
| | V9L | >100 | ND | 8,6 | <2h |
| | I9V | 1 | 5h | ND | ND |
| | P9L | >100 | ND | 4,6 | <2h |
| | E11Q | >100 | ND | >100 | ND |
| | E10L | >100 | ND | 10 | <2h |
| | L9V | 3,1 | >6h | 1,3 | >6h |
| ENV | K9L | 1,35 | >6h | 0,7 | >6h |
| | K9L/T | 0,65 | >6h | 0,4 | >6h |
| | R9V | >100 | ND | 5 | ND |
| NEF | P10L | >100 | ND | 5 | ND |
| | A9L | >100 | ND | 7 | >6h |

Les deux peptides gagp24-212 (E9V) et po179 (L10V) ont une affinité et une capacité de stabilisation faibles (RA > 5 et DC50 < 2 heures) ; en revanche leur variants P1Y ont une affinité et une capacité de stabilisation fortes (RA < 5 et DC50 > 2 heures). L'immunogénicité des peptides E9V et L10V et de leurs variants P1Y a également été testée. Les peptides natifs ne sont pas immunogènes ; en revanche leurs variants sont immunogènes aussi bien chez les souris HHD que chez l'homme.

En outre, des CTL générés, à partir de PBMC humains, provenant de 6 donneurs différents, stimulés *in vitro* par des cellules autologues chargées avec le peptide variant E9VY, ou le peptide variant L10VY, ou par le peptide natif immunodominant S9L ont également été testés pour leur capacité à tuer des cellules RMA-HHH (cellules RMA exprimant la molécule HLA A2.1 native) infectées par un virus de la vaccine recombinant exprimant soit la protéine gag, soit la protéine pol de HIV1 (isolat LAI), ou par un virus de la vaccine contrôle, de type sauvage.

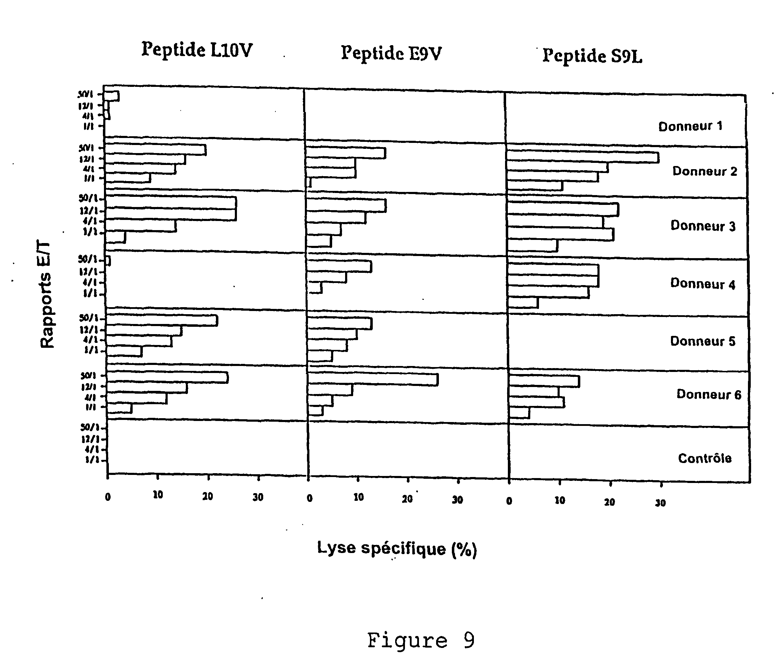

Les résultats sont illustrés par la Figure 9 (en abscisse le % de lyse spécifique ; en ordonnée le rapport cellules effectrices/cellules cibles). Ces résultats montrent que les peptides E9V et L 10V sont naturellement présentés par des cellules qui expriment HLA-A2.1 et la protéine virale dont ils sont issus (gag pour E9V et pol pour L10V).

### Epitopes de la sous-unité catalytique de la télomérase (hTERT) :

La substitution P1Y a été utilisée pour déterminer si la sous-unité catalytique de la télomérase (hTERT) possédait des épitopes capable d'induire une réponse T cytotoxique.

8 peptides ont été choisis à partir de la séquence polypeptidique de la sous-unité catalytique de la télomérase (hTERT).

L'affinité relative de ces peptides pour HLA A2.1, et la stabilité du complexe peptide/HLA A2.1 ont été déterminées comme décrit dans l'Exemple 1 ci-dessus (peptide de référence : HIVpol 589).

Les résultats sont illustrés dans le Tableau VII ci-dessous :

**Tableau VII**

| peptides natifs | Séquences | RA | DC50 |
|---|---|---|---|
| mp 530 | ILATFLAWL (SEQ ID NO: 55) | 0,8 | >6 |
| mp 534 | FLFWLMDTYV (SEQ ID NO: 56) | 0,2 | >6 |
| mp 545 | QLLRSFFHFL (SEQ ID NO: 57) | 1,8 | >6 |
| mp 797 | SLFDFFHFL (SEQ ID NO: 58) | 1,5 | >6 |
| mp 876 | FLSTLVHGV (SEQ ID NO: 59) | 1,2 | >6 |
| mhp 540 | ILAKFLHWL (SEO ID NO: 60) | 0,3 | >6 |
| mhp 572 | RLFFYRKSV (SEQ ID NO: 10) | 25,3 | <2 |
| mhp 988 | DLQVNSLQTV (SEQ ID NO: 11) | 28,6 | <2 |

Les deux peptides mhp 572 et mhp 988 ont une affinité et une capacité de stabilisation faibles (RA > 5 et DC50 < 2heures). Ces 2 peptides sont en outre communs à la sous-unité catalytique de la télomérase humaine (hTERT) et à la sous-unité catalytique de la télomérase murine (mTERT). Les variants P1Y de ces 2 peptides, résultant de la substitution de l'acide aminé N-terminal par une tyrosine, ont été préparés, et leur affinité relative pour HLA A2.1, et la stabilité du complexe peptide/HLA A2.1 ont été déterminées.

Les résultats, illustrés dans le Tableau VIII ci-dessous, montrent que ces variants mhp 572Y1 et mhp 988Y1 ont une affinité et une capacité de stabilisation fortes (RA < 5 et DC50 > 2 heures).

**Tableau VIII**

| peptides modifiés | Séquences | RA | DC50 |
|---|---|---|---|
| mhp 572Y1 | YLFFYRKSV (SEQ ID NO: 61) | 2,2 | 5 |
| mph 988Y1 | YLQVNSLQTV (SEQ ID NO: 62) | 2,1 | >6 |

Des CTL générés à partir de PBMC humains, provenant de donneurs sains, stimulés *in vitro* par des cellules dendritiques autologues chargées avec le peptide variant mhp 572Y1, ou le peptide variant mhp 988Y1 ont été testés pour leur capacité à tuer des cellules tumorales humaines HLA A2.1+/hTERT+ : U266 [VONDERHEIDE, Immunity, 10, 11, (1999)], ou HSS HLA A2.1-/hTERT+ : [VONDERHEIDE, Immunity, 10, 11, (1999)].

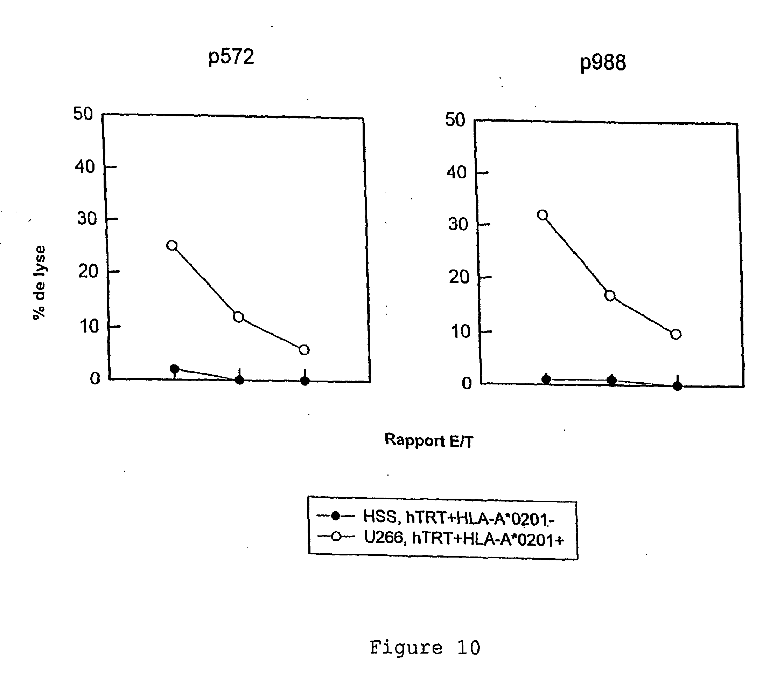

Les résultats (% de lyse en fonction du rapport cellules effectrices/cellules cibles ; rapports E/T : 40/1 ; 20/1 ; 10/1) sont illustrés par la Figure 10 : on n'observe aucune lyse pour les cellules tumorales HSS qui n'expriment pas HLA A2.1 ; en revanche, on observe une lyse importante dans le cas des cellules tumorales U266 qui expriment à la fois la sous-unité catalytique de la télomérase (hTERT) et HLA A2.1.

Ces résultats montrent que les peptides mhp 572 et mhp 988 sont naturellement présentés par les cellules tumorales humaines, et que des dérivés immunogènes de ces peptides sont potentiellement utilisables en immunothérapie anti-tumorale.

### EXEMPLE 6 : ACTIVITE ANTI-TUMORALE DES VARIANTS P1Y

### I. dérivés de l'épitope de la sous-unité catalytique de la télomérase (hTERT).

Des variants P1Y (mhp 572Y1 et mhp 988Y1), obtenus tel que décrit à l'exemple 2, sont testés pour leur capacité à induire in vivo une réponse anti-tumorale protectrice avec des peptides immunodominants (mp 797 et mp 545).

Dix souris HHD générées tel que décrit à l'exemple 1 sont vaccinées, à raison de deux injections à deux semaines d'intervalle, avec ces différents peptides synthétisés par Synt:em (Nîmes, France).

Sept jours après la dernière injection, les souris vaccinées avec les peptides mhp 572Y1, mhp 988Y1, mp 797 et mp 545 sont greffées avec des cellules tumorales EL-4/HHD [PASCOLO et al., J. Exp. Med., 185, 2043-2051 (1997)].

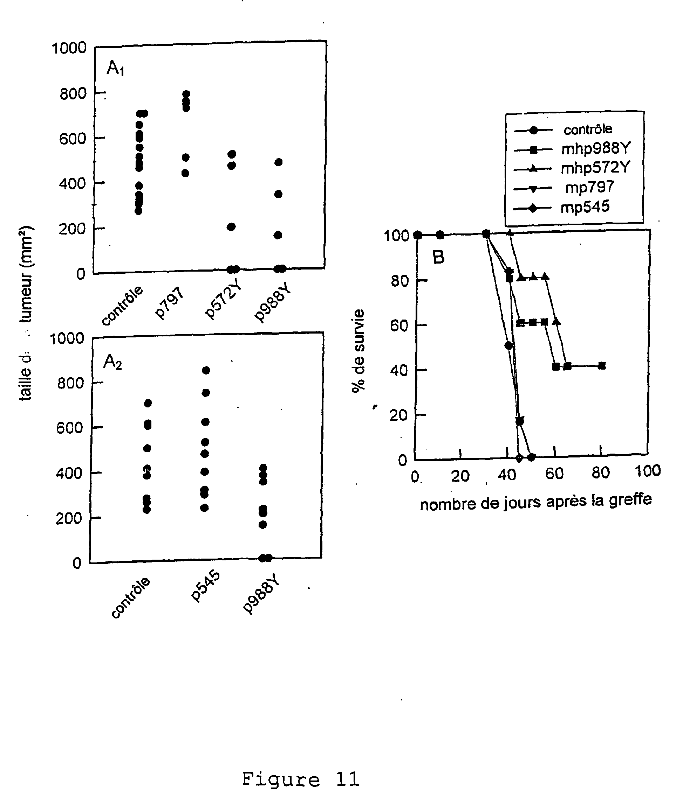

L'efficacité de la protection anti-tumorale est évaluée en mesurant la taille des tumeurs 28 jours après leur implantation (Figures 11A₁ et 11A₂ : taille des tumeurs en fonction des peptides utilisés pour la vaccination) et par la survie des souris greffées (Figure 11B : % de survie en fonction du nombre de jours après l'implantation des cellules tumorales).

Les résultats sont illustrés aux Figures 11A₁, 11A₂ et 11B : on observe qu'à J28, les tumeurs mesurent 490±144 mm² et 653±148 mm² respectivement chez les souris non traitées (contrôle) et traitées avec le peptide mp 797, 421±170 mm² et 489±209 mm² respectivement chez les souris non traitées (contrôle) et traitées avec le peptide mp 545, mais que dans le lot de souris vaccinées avec les peptides mhp 572Y1 et mhp 988Y1, les tumeurs mesurent respectivement 232±244 mm² et 190±207 mm² (Figure 11A₁) ou 213±160 mm² (Figure 11A₂), et que 4 souris vaccinées avec mhp 572Y1 et mhp 988Y1 respectivement ne présentent pas de tumeurs à J28.

Toutes les souris non vaccinées meurent à J50 (Figure 11B : ●).

Pour les souris vaccinées avec les peptides mp 797 et mp 545 (Figure 11B : ▼ et ◆), on observe une mortalité à partir de J40 et la dernière souris meurt à J50.

La mortalité est significativement réduite dans le lot de souris vaccinées avec mhp 572Y1 et mhp 988Y1 (Figure 11B : ▲ et ■). Elle apparaît à J40 et 4 souris (40%) sont encore en vie à J80 (Figure 11B : ▲, ■).

### II. dérivés de l'épitope HER-2/neu.

Des variants P1Y (HER-2/neu 650Y1, HER-2/neu 402Y1), obtenus tel que décrit à l'exemple 2, sont testés pour leur capacité à induire *in vivo* une réponse anti-tumorale protectrice avec des peptides immunodominants (HER-2/neu 369, HER-2/neu 48).

Dix souris HHD générées tel que décrit à l'exemple 1 sont vaccinées, à raison de deux injections à deux semaines d'intervalle, avec ces différents peptides synthétisés par Synt:em (Nîmes, France).

Des cellules HER-2/HHD/neu sont obtenues en transfectant les cellules tumorales EL-4/HHD avec l'ADNc codant pour la molécule HER-2/neu

Sept jours après la dernière injection, les souris vaccinées avec les peptides HER-2/neu 650Y1, HER-2/neu 402Y1, HER-2/neu 369 et HER-2/neu 48 sont greffées avec des cellules tumorales EL-4/HHD/neu.

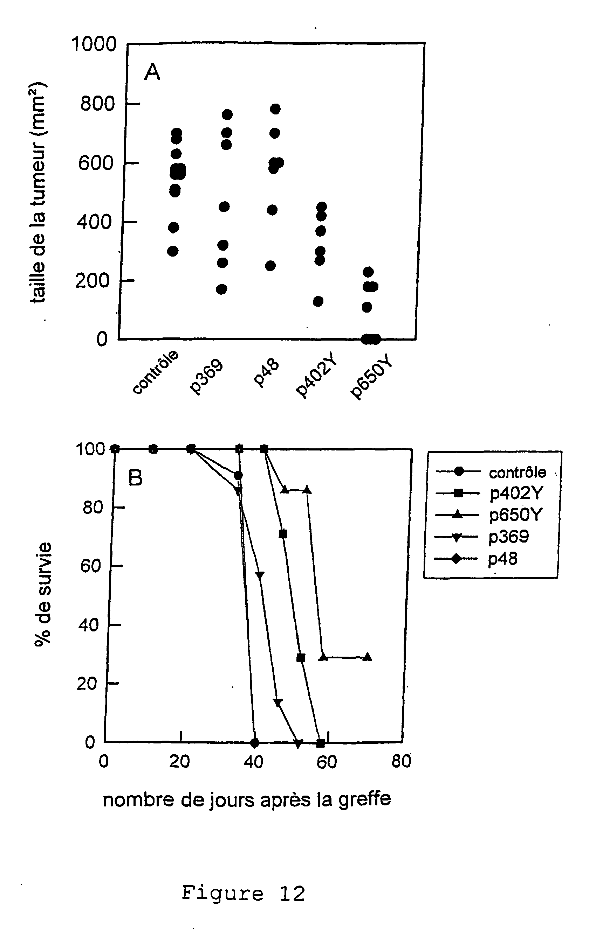

L'efficacité de la protection anti-tumorale est évaluée en mesurant la taille des tumeurs 28 jours après leur implantation (Figure 12A : taille des tumeurs en fonction des peptides utilisés pour la vaccination) et par la survie des souris greffées (Figure 12B : pourcentage de survie en fonction du nombre de jours après implantation des cellules tumorales).

Les résultats montrent qu'à J28 les tumeurs mesurent 560±93 mm², 474±234 mm² et 564±174 mm² respectivement chez les souris non traitées (contrôle) et traitées avec les peptides HER-2/neu 369 et HER-2/neu 48, mais que dans le lot de souris vaccinées avec les peptides HER-2/neu 402Y1 et HER-2/neu 650Y1, les tumeurs mesurent 323±116 mm² et. 100±99 mm², respectivement, et que 4 souris vaccinées avec HER-2/neu 650Y1 ne présentent pas de tumeurs à J28.

Toutes les souris non vaccinées meurent à J40 (Figure 12B : ●).

Pour les souris vaccinées avec les peptides HER-2/neu 369 et HER-2/neu 48 (Figure 12B : ▼ et ◆), la mortalité n'apparaît qu'à J32 et la dernière souris meurt à J52.

La mortalité est significativement réduite dans le lot de souris vaccinées avec HER-2/neu 402Y1 et HER-2/neu 650Y1 (Figure 12B : ■ et ▲). Elle n'est observée qu'à partir de J46 et 3 souris (30%) sont encore en vie à J70 (Figure 12B : ▲).

L'ensemble de ces résultats démontre que seules les formes substituées en P1 avec une tyrosine de peptides sous-dominants/cryptiques (HER-2/neu 650Y1, HER-2/neu 402Y1, mhp 572Y1 et mhp 988Y1) sont capables de générer in vivo une réponse anti-tumorale efficace. Il semble donc intéressant d'utiliser en immunothérapie anti-tumorale des peptides sous-dominants/cryptiques sous leur forme substituée en P1 avec une tyrosine.

### EXEMPLE 7 : ACTIVITE ANTI-TUMORALE INDUITE PAR DES VACCINS ADN CODANT POUR DE MULTIPLES EPITOPES DOMINANTS ET SOUS-DOMINANTS/CRYPTIQUES DERIVES DE HER-2/neu.

Afin de palier les échecs relatifs des essais cliniques menés à ce jour exclusivement au moyen de peptides tumoraux, une nouvelle approche en immunothérapie antitumorale consiste à induire des réponses multispécifiques par une immunisation génétique avec une construction polyépitopique composée de huit épitopes dominants et quatre sous-dominants/cryptiques dérivés de HER-2/neu et restreints au HLA A2.1.

### Sélection des épitopes.

Les huit épitopes dominants (HER-2/neu 799, HER-2/neu 369, HER-2/neu 789, HER-2/neu 689, HER-2/neu 773, HER-2/neu 5, HER-2/neu 48 et HER-2/neu 1023) présentent une grande affinité pour HLA A2.1 (RA < 5 et DC₅₀ > 4 heures), sauf HER-2/neu 1023 qui est considéré comme ayant une affinité de liaison intermédiaire (RA > 5, DC₅₀ > 4 heures) (cf. tableau II, Exemple 1).

Les quatre épitopes sous-dominants/cryptiques (HER-2/neu 466, HER-2/neu 402, HER-2/neu 391 et HER-2/neu 650) (cf. tableau II, Exemple 1) présentent une très faible affinité de liaison et sont non-immunogènes chez les souris HHD ou chez l'homme, alors que les variants P1Y (HER-2/neu 466Y, HER-2/neu 402Y1, HER-2/neu 391Y et HER-2/neu 650Y1), obtenus tel que décrit à l'exemple 2, présentent une grande affinité pour HLA A2.1 (RA < 4 et DC₅₀ > 4 heures). Ces variants P1Y sont représentés dans le tableau IX ci-après :

**Tableau IX**

| variants P1Y | séquence | RA | DC50 (heures) |
|---|---|---|---|
| HER-2/neu 466Y1 | YLIHHNTHL (SEQ ID NO: 66) | 1,4 | 6 |
| HER-2/neu 402Y1 | YLEEITGYL (SEQ ID NO: 67) | 3,7 | 4 |
| HER-2/neu 391Y1 | YLQPEQLQV (SEQ ID NO: 68) | 1,3 | 8 |
| HER-2/neu 650Y1 | YLTSIISAV (SEQ ID NO: 69) | 0,2 | 6 |

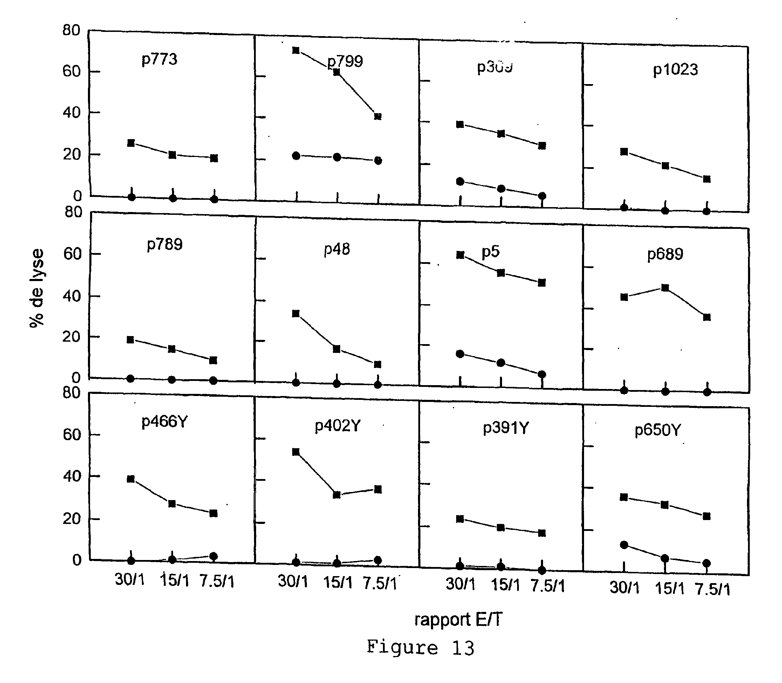

L'immunogénicité des variants P1Y et des épitopes immunodominants est testée selon le protocole décrit à l'exemple 1 :
Des souris sont vaccinées en sous-cutané avec chacun des douze peptides mentionnés ci-dessus en présence de l'épitope Th Iab dérivé de l'antigène « core » de HBV. Onze jours plus tard, leurs cellules spléniques sont restimulées *in vitro* avec le peptide à tester (1 µg) pendant six jours, et les CTL générés sont testés contre des cellules cibles RMAS-HHD chargées avec les peptides d'immunisation ou un peptide contrôle.
Les résultats (% de lyse en fonction du rapport cellules effectrices / cellules cibles) sont illustrés à la Figure 13 :
   Cellules RMAS-HHD chargées avec le peptide d'immunisation : ■
   Cellules RMAS-HHD chargées avec un peptide contrôle : ●.

Comme attendu tous ces épitopes provoquent une réponse CTL, et celles provoquées par les variants P1Y sont spécifiques pour les peptides naturels correspondants.

### Sélection d'une construction polyépitopique.

La construction polyépitopique (pet-neu) comprend une suite continue des 12 épitopes précités, à savoir :
- les huit épitopes dominants (HER-2/neu 799, HER-2/neu 369, HER-2/neu 789, HER-2/neu 689, HER-2/neu 773 HER-2/neu 5, HER-2/neu 48 et HER-2/neu 1023) décrits comme étant des cibles des lymphocytes infiltrant les tumeurs (LIT) dans les cancers du poumon, de l'ovaire, de l'estomac et du RCC, et
- les quatre variants P1Y sous-dominants/cryptiques (HER-2/neu 466Y, HER-2/neu 402Y1, HER-2/neu 391Y et HER-2/neu 650Y1) obtenus tel que décrit à l'exemple 2.

L'utilisation de la construction polyépitopique ci-dessus a été optimisée pour l'expression chez l'homme et tout codon d'initiation potentiel a été conservé aussi près que possible du site d'initiation, en particulier si des séquences Kosack sont incorporées. Une étiquette SV5-pk est ajoutée à l'extrémité 3' de la construction afin de permettre la vérification de l'expression de pet-neu dans les cellules COS transfectées (expression de l'anticorps anti-PK de 14 acides aminés).

Un plasmide de 3,0 kb, Vax1 (Invitrogen) est sélectionné, dans lequel les séquences non nécessaires à la réplication dans *E. coli* ou pour l'expression de la protéine recombinante dans les cellules de mammifère ont été retirées pour limiter les séquences d'ADN homologues au génome humain, et ce afin de minimiser la possibilité d'intégration chromosomique. Ce plasmide comporte le gène de résistance à la kanamycine, plutôt qu'à l'ampicilline, dans la mesure où les aminoglycosides sont moins susceptibles de provoquer une réponse allergique chez l'homme. L'expression est dirigée par les séquences promoteurs-activatrices du cytomégalovirus humain (CMV). Une terminaison de la transcription et une polyadénylation de l'ARNm efficaces sont obtenues en utilisant le signal de polyadénylation de l'hormone de croissance bovine.

L'ADN de pet-neu est synthétisé et cloné dans le vecteur pVax1 (Vax1/pet-neu).

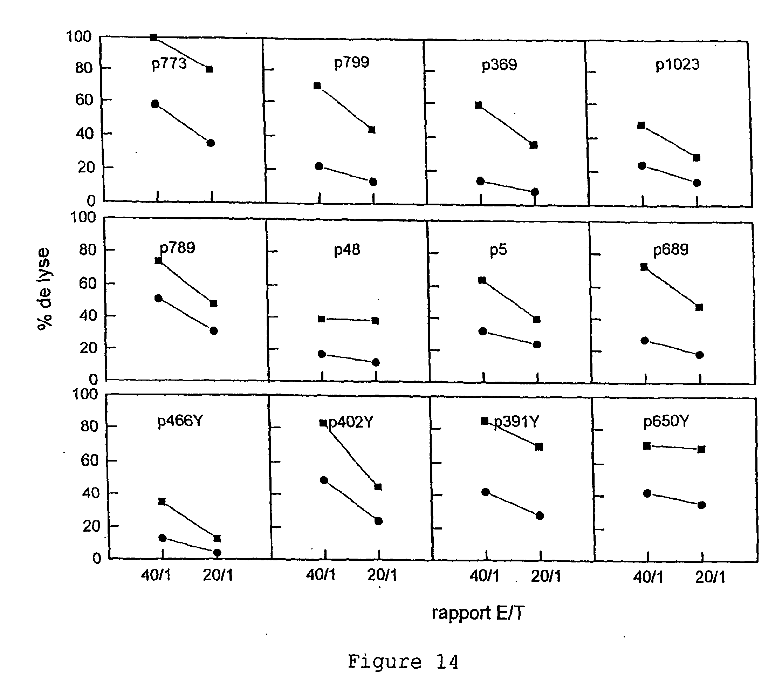

La construction polyépitopique telle que décrite ci-dessus présente les propriétés suivantes :
a) elle permet l'apprêtage de chaque épitope à son extrémité C-terminale, et
b) elle ne crée pas de nouveaux peptides de jonction avec une affinité élevée pour la molécule HLA A2.1.
L'apprêtage à l'extrémité C-terminale est évaluée par deux modèles de prédiction de clivage du protéasome ( netChop1.0, www.cbs.dtu.dk/services/NetChopPAPROC, www.uni-tuebingen.de/ uni:bcm/kuttler/links/html). Une prédiction de clivage par deux modèles est nécessaire pour considérer qu'un épitope est apprêté. L'affinité des nouveaux peptides de jonction est évaluée comme précisé à l'exemple 1 par le modèle de prédiction BIMAS [PARKER et al., J. Immunol., 152, 163, (1994)]. Parmi les différents arrangements évalués, l'arrangement de la Figure 14, correspondant à SEQ ID NO: 70, a été choisi car il répond mieux aux deux propriétés précisées ci-dessus. Les épitopes HER-2/neu 773, HER-2/neu 1023, HER-2/neu 5, HER-2/neu 466Y, HER-2/neu 391Y, HER-2/neu 650Y1 sont prédits comme étant apprêtés et seulement cinq nouveaux peptides de jonction avec une affinité élevée pour la molécule HLA A2.1 pourraient être générés (p67₉, p94₉, p17₉, p91₁₀, p63₁₀) ; ces peptides sont définis par leur position dans la séquence pet-neu (positions : 67, 94, 17, 91 et 63) et leur longueur (9 ou 10 acides aminés).

### I. Immunogénicité de Vax1/pet-neu chez des souris transgéniques HHD.

### A. Capacité de Vax1/pet-neu d'induire in vivo des CTL spécifiques.

L'immunogénicité de Vax1/pet-neu est testée tel que décrit à l'exemple 1 :

Des souris HHD, générées tel que décrit à l'exemple 1, sont immunisées en intramusculaire à raison de deux injections à 15 jours d'intervalle avec Vax1/pet-neu (150 µg). Une semaine après la dernière immunisation, leurs cellules spléniques sont restimulées *in vitro* avec des cellules B activées par le LPS et chargées avec chacun des douze peptides.

La cytotoxicité est évaluée à l'aide de cellules RMAS-HHD (cellules cibles) chargées avec le peptide correspondant à celui avec lequel les cellules B ont été chargées ou avec un peptide contrôle.

Les résultats provenant d'une souris HHD immunisée prise au hasard sont présentés à la Figure 14 (% de lyse en fonction du rapport cellules effectrices / cellules cibles).
Cellules RMAS-HHD chargées avec le peptide utilisé pour les cellules B : ■
Cellules RMAS-HHD chargées avec un peptide contrôle : ●.

Les CTL tuent les cellules RMAS-HHD chargées avec chacun des douze peptides, bien que pour des CTL spécifiques de certains peptides, une lyse des cellules cibles RMAS-HHD chargées avec un peptide contrôle est observée (lyse non spécifique).

Les lyses spécifiques les plus importantes (30% au-dessus du bruit de fond) sont obtenues avec HER-2/neu 773, HER-2/neu 799, HER-2/neu 369, HER-2/neu 689, HER-2/neu 402Y1 et HER-2/neu 391Y.

### B. Capacité des CTL à reconnaître HER-2/neu endogène.

Les CTL induits chez des souris HHD vaccinées avec Vax1/pet-neu, sont testés pour leur capacité à reconnaître spécifiquement HER-2/neu endogène.

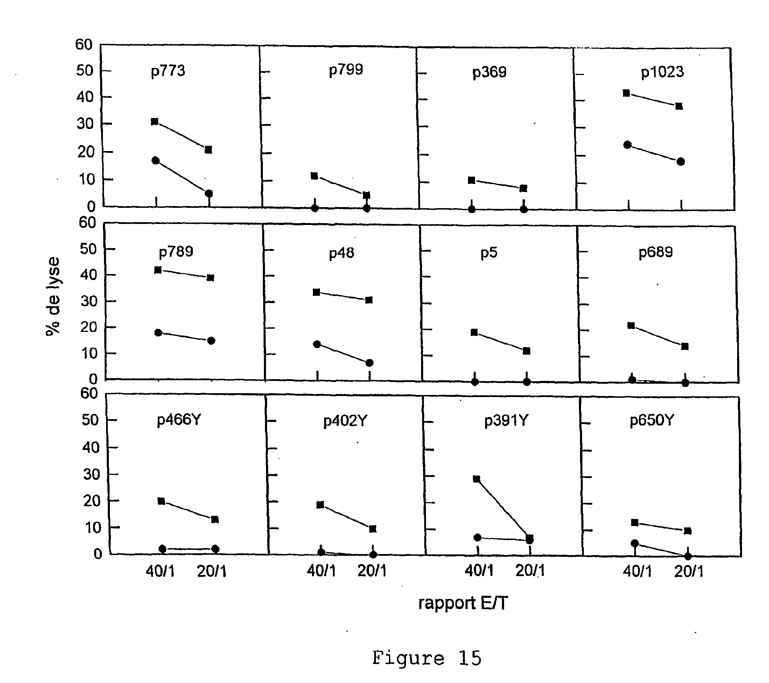

Les résultats sont présentés à la Figure 15 (% de lyse en fonction du rapport cellules effectrices (E) / cellules cibles (T)).

Les CTL sont induits comme décrit en A ci-dessus et sont testés contre les cellules cibles EL-4/HHD (●) référencées à l'exemple 6 et les cellules cibles EL-4/HHD/neu (■) obtenues en transfectant les cellules EL-4/HHD avec l'ADNc codant pour HER-2/neu.

Les CTL spécifiques des douze peptides de pet-neu reconnaissent et tuent les cibles EL-4/HHD/neu exprimant HER-2/neu mais pas les cibles EL-4/HHD utilisées comme contrôle négatif.

Pour les trois souris testées, une lyse spécifique plus importante (> 20% au-dessus du bruit de fond) est obtenue pour HER-2/neu 1023, HER-2/neu 789, HER-2/neu 48, HER-2/neu 689, HER-2/neu 466Y, HER-2/neu 402Y1 et HER-2/neu 391Y.

### C. Facteur en cause dans l'induction des CTL.

L'induction des CTL nécessite soit la sensibilisation avec Vax1/pet-neu ou bien résulte de la répétition des stimulations *in vitro* des cellules spléniques naïves.

L'immunogénicité de Vax1/pet-neu est testée tel que décrit à l'exemple 1 :

Six souris sont immunisées avec soit la construction Vax1/pet-neu soit le vecteur Vax1. Leurs cellules spléniques sont stimulées *in vitro* de façon répétitive avec les cellules B activées par le LPS et chargées avec chaque peptide à tester (cellules effectrices).

Les cellules cibles utilisées pour étudier la spécificité des CTL vis-à-vis de HER-2/neu sont des cellules RMAS-HHD et les cellules EL-4/HHD/neu obtenues tel que décrit à l'exemple 6.

La cytotoxicité est testée après la troisième stimulation *in vitro.* Les cellules RMAS-HHD, chargées avec le peptide correspondant à celui avec lequel les cellules B ont été chargées ou avec un peptide contrôle, et les cellules EL-4/HHD/neu sont utilisées comme cellules cibles.

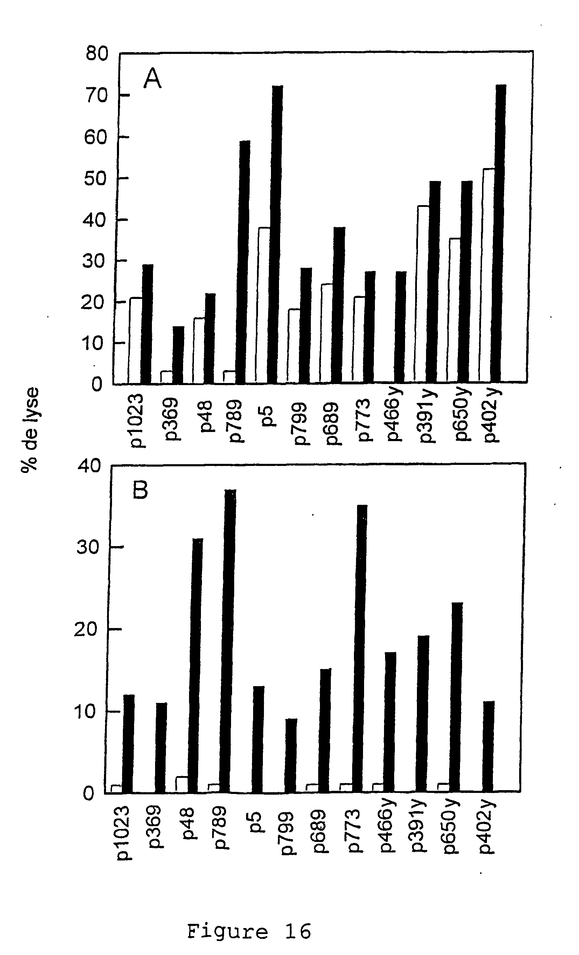

Les résultats sont présentés à la Figure 16 [% de lyse spécifique (la lyse des cellules cibles chargées avec un peptide contrôle est déduite) après une immunisation avec Vax1 (□) ou Vax1/pet-neu (■)].

Les cellules spléniques sensibilisées avec Vax1/pet-neu conduisent à des CTL spécifiques pour les douze peptides. De façon surprenante, les CTL contre la majorité des peptides, sauf HER-2/neu 369 et HER-2/neu 789, sont générés à partir des cellules spléniques sensibilisées avec le vecteur Vax1. Pour quelques peptides (EIER-2/neu 1023, HER-2/neu 48, HER-2/neu 799, HER-2/neu 773, HER-2/neu 391Y), la cytotoxicité des CTL induits à partir des cellules spléniques sensibilisées avec Vax1 est presque aussi élevée que la cytotoxicité des CTL induits à partir des cellules spléniques sensibilisées avec Vax1/pet-neu (Figure 16A). Cela démontre que les stimulations *in vitro* répétitives sont suffisantes pour déclencher l'induction de CTL.

Cependant, ces CTL bien qu'ils éliminent des cibles chargées avec les peptides sont incapables de reconnaître et d'éliminer des cellules cibles exprimant HER-2/neu endogène (EL-4/HHD/neu) contrairement aux CTL induits à partir des cellules spléniques sensibilisées avec Vax1/pet-neu (Figure 16B).

L'ensemble de ces résultats démontre que les CTL spécifiques de HER-2/neu capables d'éliminer efficacement les cellules tumorales exprimant l'antigène sont générés par une vaccination Vax1/pet-neu.

### II. La vaccination Vax1/pet-neu induit in vivo une immunité anti-tumorale.

Les résultats obtenus *in vitro* ont conduit à rechercher si la vaccination Vax1/pet-neu induit *in vivo* une immunité anti-tumorale protectrice.

### A. Effet sur la croissance des tumeurs et spécificité de la protection anti-tumorale.

Un sous-clone des cellules EL-4/HHD/neu, générées tel que décrit à l'exemple 6, extrêmement tumorigénique est obtenu après trois sélections *in vivo* dans les souris HHD. Il exprime HHD et HER-2/neu au même niveau que les cellules parentales.

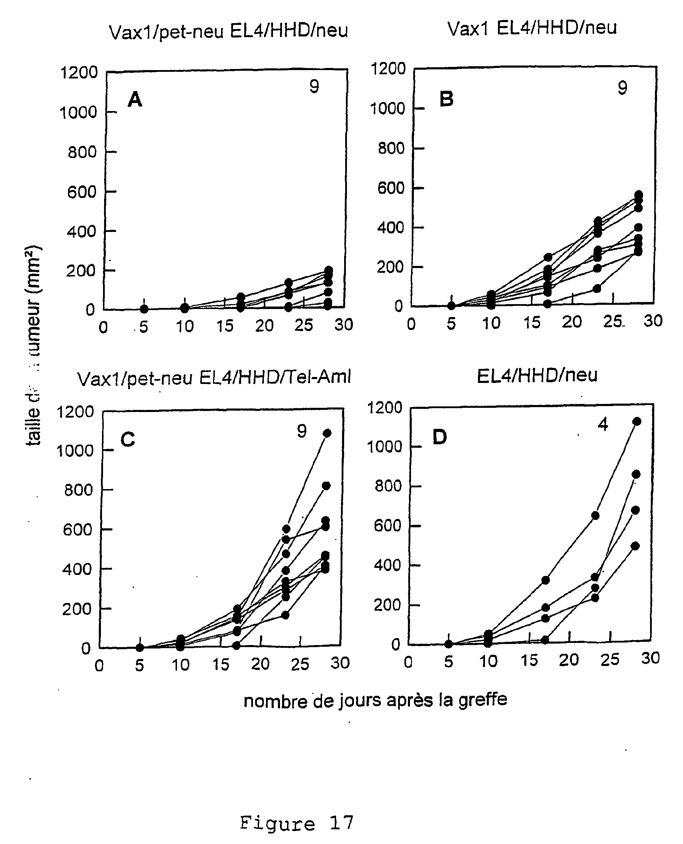

Des souris HHD, générées tel que décrit à l'exemple 1, sont immunisées à raison de 2 injections à deux semaines d'intervalle avec la construction Vax1/pet-neu (Figures 17A et 17C) ou avec le vecteur Vax1 vide (Figure 17B) ou ne sont pas immunisées (Figure 17D).

Une semaine après la dernière immunisation, les souris ci-dessus sont greffées avec 2x10⁴ cellules EL-4/HHD/neu (Figures 17A, 17B et 17D) ou avec des cellules EL-4/HHD/Tel-Aml, obtenues en transfectant les cellules EL-4/HHD avec l'ADNc codant pour la protéine recombinante Tel-Aml, pour tester in *vivo* la spécificité de l'immunité protectrice anti-tumorale.

La croissance des tumeurs est évaluée tous les 5 à 7 jours jusqu'à J28 lorsque toutes les souris non vaccinées sont mortes et que les souris des groupes restants sont suivies pour leur mortalité.

Les résultats sont présentés dans la Figure 17 (taille des tumeurs en fonction du nombre de jours après l'implantation des cellules tumorales).

Toutes les souris contrôle non vaccinées (Figure 17D) ou vaccinées avec Vax1 (Figure 17B) développent des tumeurs qui apparaissent à J17 et grossissent très rapidement. A J28 les tumeurs mesurent 772±268 mm² et 404±121 mm² chez les souris contrôle et traitées avec Vax1 respectivement (p=0,04).

Au contraire, dans le lot de souris vaccinées avec Vax1/pet-neu (Figure 17A), 2 souris sur 9 ne présentent pas de tumeurs à J28, sinon les tumeurs apparaissent à J23 et grossissent lentement. La taille des tumeurs est 116±66 mm² (p=0,0001 par comparaison avec soit les souris non traitées ou les souris traitées avec Vaxl).

Cette immunité protectrice anti-tumorale in vivo est spécifique de HER-2/neu du fait que les souris vaccinées avec Vax1/pet-neu ne sont pas protégées contre la tumeur EL-4/HHD/Tel-Aml (Figure 17C). En effet, toutes les souris développent des tumeurs et leur taille à J28 est 578±231 mm².

### B. Effet sur la survie des souris et spécificité de la protection anti-tumorale.

L'effet de la protection anti-tumorale induite par la vaccination Vax1/pet-neu est confirmé en examinant la survie de souris portant des tumeurs.

Des souris HHD sont vaccinées et greffées tel que décrit en A. Leur mortalité est suivie jusqu'à J55.

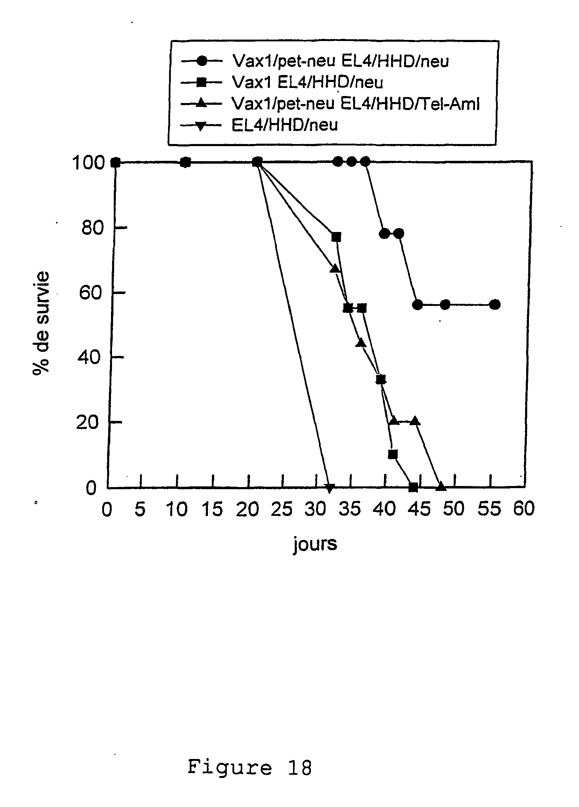

Les résultats sont présentés dans la Figure 18 (% de survie en fonction du nombre de jours après l'implantation des cellules tumorales).

Toutes les souris non vaccinées meurent à J32 (▼).

Pour les souris vaccinées avec Vax1 (■), la mortalité commence à J32 et la dernière souris meurt à J42 (p=0,04 par comparaison avec les souris non vaccinées).

La mortalité est significativement réduite dans le lot de souris vaccinées avec Vax1/pet-neu (●). Elle commence à J39 et 5 souris (56%) sont encore en vie à J55 (p=0,0008 par comparaison aux souris non traitées et traitées avec Vax1).

Cette immunité protectrice est spécifique pour HER-2/neu du fait que la mortalité des souris traitées avec Vax1/pet-neu et greffées avec la tumeur EL4/HHD/Tel-Aml (▲) est similaire à la mortalité des souris traitées avec Vax1 et greffées avec les cellules tumorales EL-4/HHD/neu (■).

## Revendications

1. Procédé d'identification d'épitopes sous-dominants/cryptiques présentés par la molécule HLA A2.1 de classe I, **caractérisé en ce qu'**il comprend au moins les étapes suivantes :
a) le choix, à partir de la séquence d'une protéine vis-à-vis de laquelle on souhaite induire une réponse cytotoxique T, d'au moins une séquence peptidique de 8 à 11 acides aminés qui possède tout ou partie du motif d'ancrage primaire de la molécule HLA A2.1, et telle que le complexe de ce peptide avec la molécule HLA A2.1 possède une DC50 inférieure à 2 heures ;
b) la préparation, pour chaque séquence sélectionnée, d'un peptide variant dérivé de ladite séquence par substitution de l'acide aminé N-terminal par un résidu tyrosine ;
c) la détermination de l'immunogénicité de chaque peptide variant obtenu à l'étape b) par la sélection, parmi ceux-ci, de chaque peptide immunogène générant une réponse CTL spécifique vis-à-vis de cellules cibles exprimant la protéine dont est issue la séquence peptidique choisie à l'étape a), et l'identification de la séquence peptidique dont dérive ledit peptide immunogène.

2. Procédé selon la revendication 1, **caractérisé en ce que** la séquence peptidique choisie à l'étape a) correspond à un peptide qui possède une affinité relative pour la molécule HLA A2.1 supérieure à 5, de préférence supérieure à 10 par rapport à HIVpol 589 (SEQ ID NO : 3).

3. Procédé de détermination d'une séquence d'un peptide immunogène présenté par la molécule HLA A2.1 de classe I, capable d'induire une réponse cytotoxique T dirigée contre un épitope non immunogène d'une protéine cible, ledit procédé étant **caractérisé en ce qu'**il comprend au moins les étapes suivantes :
a) l'identification, par un procédé selon la revendication 1 ou la revendication 2, d'un épitope sous-dominant/cryptique de la protéine vis-à-vis de laquelle on souhaite induire une réponse cytotoxique T, et
b) la modification de la séquence dudit épitope sous-dominant/cryptique, par substitution de l'acide aminé N-terminal par un résidu tyrosine.

4. Peptide immunogène **caractérisé en ce qu'**il est dérivé, par substitution de l'acide aminé N-terminal par un résidu tyrosine, d'un épitope sous-dominant/cryptique présenté par HLA A2.1 choisi parmi :
le peptide HER-2/neu 650 : PLTSIISAV (SEQ ID NO: 4)
le peptide HER-2/neu 466 : ALIHHNTHL (SEQ ID NO: 5)
le peptide HER-2/neu 402 : TLEEITGYL (SEQ ID NO: 6)
le peptide HER-2/neu 391: PLQPEQLQV (SEQ ID NO: 7)
le peptide gagp24-212 : EMMTACQGV (SEQ ID NO: 8)
le peptide pol79 : LLDTGADDTV (SEQ ID NO: 9)
le peptide mhp 572 : RLFFYRKSV (SEQ ID NO: 10)
le peptide mhp 988 : DLQVNSLQTV (SEQ ID NO: 11)

5. Épitope sous-dominant/cryptique présenté par HLA A2.1, **caractérisé en ce qu'**il s'agit du peptide mhp 988 : DLQVNSLQTV (SEQ ID NO: 11).

6. Composition comprenant au moins un épitope peptidique immunogène selon la revendication 4.

7. Composition selon la revendication 6, **caractérisée en ce qu'**elle comprend en outre un ou plusieurs autre (s) épitope (s) immunogène(s).

8. Composition selon une quelconque des revendications 6 ou 7, **caractérisée en ce qu'**elle comprend un polypeptide chimérique comprenant une ou plusieurs copies d'un épitope peptidique immunogène selon la revendication 4.

9. Composition selon la revendication 8, **caractérisée en ce que** ledit polypeptide chimérique comprend en outre une ou plusieurs copies d'un ou plusieurs autre(s) épitope(s) immunogène (s).

10. Molécule d'acide nucléique codant un polypeptide chimérique selon une quelconque des revendications 8 ou 9.

11. Molécule d'acide nucléique selon la revendication 10 **caractérisée en ce qu'**elle comprend une séquence codant :
l'épitope HER-2/neu 650Y1 : YLTSIISAV (SEQ ID NO: 69)
l'épitope HER-2/neu 466Y : YLIHHNTHL (SEQ ID NO: 66)
l'épitope HER-2/neu 402Y1 : YLEEITGYL (SEQ ID NO: 67)
l'épitope HER-2/neu 391Y : YLQPEQLQV (SEQ ID NO: 68)

12. Molécule d'acide nucléique selon la revendication 11 **caractérisée en ce qu'**elle comprend en outre une séquence codant :
l'épitope HER-2/neu 799 : QLMPYGCLL (SEQ ID NO: 27)
l'épitope HER-2/neu 369 : KIFGSLAFL (SEQ ID NO: 28)
l'épitope HER-2/neu 789 : CLTSTVQLV (SEQ ID NO: 24)
l'épitope HER-2/neu 689 : RLLQETELV (SEQ ID NO: 65)
l'épitope HER-2/neu 773 : VMAGVGSPYV (SEQ ID NO: 31)
l'épitope HER-2/neu 5 : ALCRWGLLL (SEQ ID NO: 30)
l'épitope HER-2/neu 48 : HLYQGCQVV (SEQ ID NO: 25)
l'épitope HER-2/neu 1023 : YLVPQQGFFC (SEQ ID NO: 64)

13. Médicament comprenant, en tant que principe actif, au moins un épitope peptidique immunogène selon la revendication 4, ou au moins une composition selon une quelconque des revendications 6 à 8, ou au moins une molécule d'acide nucléique selon les revendications 10 à 12.

14. Médicament selon la revendication 13, **caractérisé en ce qu'**il est destiné à l'immunothérapie antivirale ou antitumorale, préventive ou curative.

15. Médicament selon une quelconque des revendications 13 ou 14, **caractérisé en ce qu'**il s'agit d'un vaccin.

16. Utilisation d'un épitope peptidique selon la revendication 4, d'une composition selon une quelconque des revendications 6 à 8, ou d'une molécule d'acide nucléique selon les revendications 10 à 12, pour l'obtention d'un médicament destiné à l'immunothérapie antivirale ou antitumorale.

## Claims

1. Method of identifying subdominant/cryptic epitopes presented by the class I HLA A2.1 molecule, **characterised in that** it comprises at least the following steps:
a) choosing, from the sequence of a protein to which it is desired to induce a cytotoxic T response, at least one peptide sequence having from 8 to 11 amino acids which possesses all or part of the primary anchor motif of the HLA A2.1 molecule, and such that the complex of that peptide with the HLA A2.1 molecule has a DC50 of less than 2 hours;
b) preparing, for each selected sequence, a variant peptide derived from said sequence by substitution of the N-terminal amino acid by a tyrosine residue;
c) determining the immunogenicity of each variant peptide obtained in step b) by selecting, from those variant peptides, each immunogenic peptide that generates a specific CTL response against target cells expressing the protein from which the peptide sequence chosen in step a) is derived, and identifying the peptide sequence from which said immunogenic peptide is derived.

2. Method according to claim 1, **characterised in that** the peptide sequence chosen in step a) corresponds to a peptide which has a relative affinity for the HLA A2.1 molecule greater than 5, preferably greater than 10, relative to HIVpol 589 (SEQ ID NO: 3).

3. Method for determining a sequence of an immunogenic peptide presented by the class I HLA A2.1 molecule, capable of inducing a cytotoxic T response directed against a non-immunogenic epitope of a target protein, said method being **characterised in that** it comprises at least the following steps:
a) identifying, by a method according to claim 1 or claim 2, a subdominant/cryptic epitope of the protein to which it is desired to induce a cytotoxic T response, and
b) modifying the sequence of said subdominant/cryptic epitope by substitution of the N-terminal amino acid by a tyrosine residue.

4. Immunogenic peptide, **characterised in that** it is derived, by substitution of the N-terminal amino acid by a tyrosine residue, from a subdominant/cryptic epitope presented by HLA A2.1 selected from:
the peptide HER-2/neu 650 : PLTSIISAV (SEQ ID NO: 4)
the peptide HER-2/neu 466 : ALIHHNTHL (SEQ ID NO: 5)
the peptide HER-2/neu 402 : TLEEITGYL (SEQ ID NO: 6)
the peptide HER-2/neu 391 : PLQPEQLQV (SEQ ID NO: 7)
the peptide gagp24-212 : EMMTACQGV (SEQ ID NO: 8)
the peptide pol79 : LLDTGADDTV (SEQ ID NO: 9)
the peptide mhp 572 : RLFFYRKSV (SEQ ID NO: 10)
the peptide mhp 988: DLQVNSLQTV (SEQ ID NO: 11).

5. Subdominant/cryptic epitope presented by HLA A2.1, **characterised in that** it is the peptide mhp 988 : DLQVNSLQTV (SEQ ID NO: 11).

6. Composition comprising at least one immunogenic peptide epitope according to claim 4.

7. Composition according to claim 6, **characterised in that** it further comprises one or more other immunogenic epitope(s).

8. Composition according to either claim 6 or claim 7, **characterised in that** it comprises a chimeric polypeptide comprising one or more copies of an immunogenic peptide epitope according to claim 4.

9. Composition according to claim 8, **characterised in that** said chimeric polypeptide further comprises one or more copies of one or more other immunogenic epitope(s).

10. Nucleic acid molecule coding for a chimeric polypeptide according to either claim 8 or claim 9.

11. Nucleic acid molecule according to claim 10, **characterised in that** it comprises a sequence coding for:
the epitope HER-2/neu 650Y1 : YLTSIISAV (SEQ ID NO: 69)
the epitope HER-2/neu 466Y : YLIHHNTHL (SEQ ID NO: 66)
the epitope HER-2/neu 402Y1 : YLEEITGYL (SEQ ID NO: 67)
the epitope HER-2/neu 391Y : YLQPEQLQV (SEQ ID NO: 68).

12. Nucleic acid molecule according to claim 11, **characterised in that** it further comprises a sequence coding for:
the epitope HER-2/neu 799 : QLMPYGCLL (SEQ ID NO: 27)
the epitope HER-2/neu 369 : KIFGSLAFL (SEQ ID NO: 28)
the epitope HER-2/neu 789 : CLTSTVQLV (SEQ ID NO: 24)
the epitope HER-2/neu 689 : RLLQETELV (SEQ ID NO: 65)
the epitope HER-2/neu 773 : VMAGVGSPYV (SEQ ID NO: 31)
the epitope HER-2/neu 5 : ALCRWGLLL (SEQ ID NO: 30)
the epitope HER-2/neu 48 : HLYQGCQVV (SEQ ID NO: 25)
the epitope HER-2/neu 1023 : YLVPQQGFFC (SEQ ID NO: 64).

13. Medicament comprising, as active ingredient, at least one immunogenic peptide epitope according to claim 4, or at least one composition according to any one of claims 6 to 8, or at least one nucleic acid molecule according to any one of claims 10 to 12.

14. Medicament according to claim 13, **characterised in that** it is intended for preventive or curative antiviral or antitumour immunotherapy.

15. Medicament according to either claim 13 or claim 14, **characterised in that** it is a vaccine.

16. Use of a peptide epitope according to claim 4, of a composition according to any one of claims 6 to 8,or of a nucleic acid molecule according to claims 10 to 12, in the preparation of a medicament for antiviral or antitumour immunotherapy.

## Patentansprüche

1. Verfahren zur Identifizierung von subdominanten/kryptischen Epitopen, die durch das Klasse I-Molekül HLA A2.1 präsentiert werden, **dadurch gekennzeichnet, dass** es wenigstens die folgenden Stufen umfasst:
a) Auswahl aus der Sequenz eines Proteins, gegen das man eine cytotoxische T-Antwort induzieren möchte, wenigstens einer Peptidsequenz mit 8 bis 11 Aminosäuren, die das primäre Verankerungsmotiv des Moleküls HLA A2.1 ganz oder einen Teil davon besitzt und eine solche ist,mdd dass der Komplex dieses Peptids mit dem Molekül HLA A2.1 eine DC50 von unter 2 Stunden besitzt;
b) für jede ausgewählte Sequenz Herstellung eines varianten Peptids, das von der genannten Sequenz durch Substitution der N-terminalen Aminosäure durch einen Tyrosinrest abgeleitet wird;
c) Bestimmung der Immunogenität jedes varianten Peptids, das in Stufe b) erhalten wurde, durch Selektion jedes immunogenen Peptids unter diesen, das eine spezifische CTL-Antwort gegenüber Targetzellen erzeugt, die das Protein exprimieren, das aus der in Stufe a) ausgewählten Peptidsequenz stammt, und Identifizierung der Peptidsequenz, von der das immunogene Peptid abgeleitet ist.

2. Verfahren nach Anspruch 1, **dadurch gekennzeichnet, dass** die in Stufe a) ausgewählte Peptidsequenz einem Peptid entspricht, das verglichen mit HIVpol 589 (SEQ ID NO: 3) eine relative Affinität für das Molekül HLA A2.1 von über 5, vorzugsweise über 10, besitzt.

3. Verfahren zur Bestimmung einer Sequenz eines immunogenen Peptids, das durch das Klasse I-Molekül HLA A2.1 präsentiert wird, die fähig ist, eine cytotoxische T-Antwort, die gegen ein nicht immunogenes Epitop eines Targetproteins gerichtet ist, zu induzieren, wobei das Verfahren **dadurch gekennzeichnet ist, dass** es wenigstens die folgenden Stufen umfasst:
a) Identifizierung eines subdominanten/kryptischen Epitops des Proteins, gegen das man eine cytotoxische T-Antwort induzieren möchte, durch ein Verfahren gemäß Anspruch 1 oder Anspruch 2, und
b) Modifikation der Sequenz des genannten subdominanten/kryptischen Epitops durch Substitution der N-terminalen Aminosäure durch einen Tyrosinrest.

4. Immunogenes Peptid, **dadurch gekennzeichnet, dass** es durch Substitution der N-terminalen Aminosäure durch einen Tyrosinrest abgeleitet ist von einem subdominanten/kryptischen Epitop, das durch HLA A2.1 präsentiert wird, ausgewählt aus:
dem Peptid HER-2/neu 650: PLTSIISAV (SEQ ID NO: 4)
dem Peptid HER-2/neu 466: ALIHHNTHL (SEQ ID NO: 5)
dem Peptid HER-2/neu 402: TLEEITGYL (SEQ ID NO: 6)
dem Peptid HER-2/neu 391: PLQPEQLQV (SEQ ID NO: 7)
dem Peptid gagp24-212: EMMTACQGV (SEQ ID NO: 8)
dem Peptid po179: LLDTGADDTV (SEQ ID NO: 9)
dem Peptid mhp 572: RLFFYRKSV (SEQ ID NO: 10)
dem Peptid mhp 988: DLQVNSLQTV (SEQ ID NO: 11).

5. Subdominantes/kryptisches Epitop, das durch HLA A2.1 präsentiert wird, **dadurch gekennzeichnet, dass** es sich um das Peptid mhp 988: DLQVNSLQTV (SEQ ID NO: 11) handelt.

6. Zusammensetzung, umfassend wenigstens ein immunogenes Peptidepitop gemäß Anspruch 4.

7. Zusammensetzung nach Anspruch 6, **dadurch gekennzeichnet, dass** sie außerdem ein weiteres immunogenes Epitop oder mehrere andere immunogene Epitope umfasst.

8. Zusammensetzung nach einem der Ansprüche 6 oder 7, **dadurch gekennzeichnet, dass** sie ein chimäres Polypeptid umfasst, das eine Kopie oder mehrere Kopien eines immunogenen Peptidepitops nach Anspruch 4 umfasst.

9. Zusammensetzung nach Anspruch 8, **dadurch gekennzeichnet, dass** das chimäre Polypeptid außerdem eine Kopie oder mehrere Kopien eines anderen immunogenen Epitops oder mehrerer anderer immunogener Epitope umfasst.

10. Nucleinsäuremolekül, das für ein chimäres Polypeptid nach einem der Ansprüche 8 oder 9 codiert.

11. Nucleinsäuremolekül nach Anspruch 10, **dadurch gekennzeichnet, dass** es eine Sequenz umfasst,die codiert für
das Epitop HER-2/neu 650Y1: YLTSIISAV (SEQ ID NO: 69)
das Epitop HER-2/neu 466Y: YLIHHNTHL (SEQ ID NO: 66)
das Epitop HER-2/neu 402Y1: YLEEITGYL (SEQ ID NO: 67)
das Epitop HER-2/neu 391Y: YLQPEQLQV (SEQ ID NO: 68).

12. Nucleinsäuremolekül nach Anspruch 11, **dadurch gekennzeichnet, dass** es außerdem eine Sequenz umfasst, die codiert für:
das Epitop HER-2/neu 799: QLMPYGCLL (SEQ ID NO: 27)
das Epitop HER-2/neu 369: KIFGSLAFL (SEQ ID NO: 28)
das Epitop HER-2/neu 789: CLTSTVQLV (SEQ ID NO: 24)
das Epitop HER-2/neu 689: RLLQETELV (SEQ ID NO: 65)
das Epitop HER-2/neu 773: VMAGVGSPYV (SEQ ID NO: 31)
das Epitop HER-2/neu 5: ALCRWGLLL (SEQ ID NO: 30)
das Epitop HER-2/neu 48: HLYQGCQVV (SEQ ID NO: 25)
das Epitop HER-2/neu 1023: YLVPQQGFFC (SEQ ID NO: 64).

13. Medikament, das als Wirkstoff wenigstens ein immunogenes Peptidepitop nach Anspruch 4 oder wenigstens eine Zusammensetzung nach einem der Ansprüche 6 bis 8 oder wenigstens ein Nucleinsäuremolekül nach den Ansprüchen 10 bis 12 umfasst.

14. Medikament nach Anspruch 13, **dadurch gekennzeichnet, dass** es für die antivirale oder antitumorale, präventive oder kurative Immuntherapie bestimmt ist.

15. Medikament nach einem der Ansprüche 13 oder 14, **dadurch gekennzeichnet, dass** es sich um ein Vakzin handelt.

16. Verwendung eines Peptidepitops nach Anspruch 4, einer Zusammensetzung nach einem der Ansprüche 6 bis 8 oder eines Nucleinsäuremoleküls nach den Ansprüchen 10 bis 12 zur Herstellung eines Medikaments, das für die antivirale oder anti-tumorale Immuntherapie bestimmt ist.
